# EUROPEAN PATENT SPECIFICATION

(11) **EP 1 039 862 B1**
(45) Date of publication and mention of the grant of the patent: **21.05.2008**
(21) Application number: 98964730.0
(22) Date of filing: 14.12.1998
(51) Int. Cl.: A61B 18/14

(54) **SYSTEMS FOR ELECTROSURGICAL TREATMENT OF THE HEAD AND NECK**
SYSTEME ZUR ELEKTROCHIRURGISCHEN BEHANDLUNG VON KOPF UND NACKEN
SYSTEMES DE TRAITEMENT ELECTROCHIRURGICAL DE LA TETE ET DU COU

(30) Priority: 15.12.1997 US 990374; 02.04.1998 US 54323; 22.05.1998 US 83526; 18.08.1998 US 136079
(43) Date of publication of application: 04.10.2000
(73) Proprietor: ArthroCare Corporation, Austin, TX 78735 (US)
(72) Inventor: HOVDA, David, C., Mountain View, CA 94040 (US); ELLSBERRY, Maria, B., Fremont, CA 94539 (US); THAPLIYAL, Hira, V., Los Altos, CA 94024 (US); EGGERS, Philip, E., Dublin, OH 43017 (US)
(74) Representative: Warren, Caroline Elisabeth
(86) International application number: PCT/US1998/026624
(87) International publication number: WO 1999/030655

(56) References cited:
- WO-A-96/39914
- WO-A-97/41786
- US-A- 4 674 499
- US-A- 5 281 216
- US-A- 5 281 218
- US-A- 5 342 357

## Description

### RELATED APPLICATIONS

### BACKGROUND OF THE INVENTION

The present invention relates generally to the field of electrosurgery, and more particularly to surgical devices which employ high frequency electrical energy to treat tissue in regions of the head and neck, such as the ear, nose and throat. The present invention is particularly suited for sinus surgery and the treatment of obstructive sleep disorders.

Sinuses are the air-filled cavities insides the facial bones that open into the nasal cavities. Sinusitis is the inflammation of the mucous membranes of one or more of the paranasal sinus cavities. Sinusitis is often associated with a viral or bacterial upper respiratory infection that spreads to the sinuses. When the sinus opening becomes blocked, the cavities fill, producing deep pain and pressure. Postnasal or nasal drainage, nasal congestion with pressure, headaches, sinus infections and nasal polyps are most commonly associated with chronic sinusitis.

Treatment of mild sinusitis typically involves antibiotics, decongestants and analgesics, and is designed to prevent further complications. For more severe or chronic sinusitis, surgery is often necessary to return the nose and sinuses to normal function, particularly with patients who have undergone years of allergy treatment and still suffer from sinus blockage, or patients born with small sinuses and nasal passages. Recent developments in the field of endoscopic surgical techniques and medical devices have provided skilled physicians with instrumentation and methods to perform complicated paranasal sinus surgical procedures. Improved visualization of the nasal cavity and the paranasal sinuses, for example, has now made these anatomical areas more accessible to the endoscopic surgeon. As a result, functional endoscopic sinus surgery (FESS) has become the technique of choice in the surgical approach to sinus disease.

Another nasal symptom, runny noses (e.g., allergic rhinitis or vasomotor rhinitis), is typically caused by small shelf-like structures in the nose called turbinates. Turbinates are responsible for warming and humidifying the air passing through the nose into the lungs. When the air contains an irritant, the turbinates react to the airborne particles by swelling and pouring mucus, as if the body were trying to block and cleanse the breathing passage. For temporary relief of swollen turbinates, decongestant nasal sprays and pills are often prescribed. These measures, however, have limited effectiveness, and the long term use of such nasal sprays typically makes the problem worse. Moreover, decongestant pills may cause high blood pressure, increase the heart rate and, for some people, cause sleeplessness.

In the past several years, powered instrumentation, such as microdebrider devices and lasers, has been used to remove polyps or other swollen tissue in functional endoscopic sinus surgery. Microdebriders are disposable motorized cutters having a rotating shaft with a serrated distal tip for cutting and resecting tissue. The handle of the microdebrider is typically hollow, and it accommodates a small vacuum, which serves to aspirate debris. In this procedure, the distal tip of the shaft is endoscopically delivered through a nasal passage into the sinus cavity of a patient, and an endoscope is similarly delivered through the same or the opposite nasal passage to view the surgical site. An external motor rotates the shaft and the serrated tip, allowing the tip to cut the polyps or other tissue responsible for the sinus blockage. Once the critical blockage is cleared, aeration and drainage are reestablished and the sinuses heal and return to their normal function.

While microdebriders have been promising, these devices suffer from a number of disadvantages. For one thing, the tissue in the nasal and sinus cavities is extremely vascular, and the microdebrider severs blood vessels within this tissue, usually causing profuse bleeding that obstructs the surgeon's view of the target site. Controlling this bleeding can be difficult since the vacuuming action tends to promote hemorrhaging from blood vessels disrupted during the procedure. In addition, the microdebrider often must be removed from the nose periodically to cauterize severed blood vessels, which lengthens the procedure. Moreover, the serrated edges and other fine crevices of the microdebrider can easily become clogged with debris, which requires the surgeon to remove and clean the microdebrider during the surgery, further increasing the length of the procedure. More serious concerns, however, are that the microdebrider is not precise, and it is often difficult, during the procedure, to differentiate between the target sinus tissue, and other structures within the nose, such as cartilage, bone or cranial. Thus, the surgeon must be extremely careful to minimize damage to the cartilage and bone within the nose, and to avoid damaging nerves, such as the optic nerve.

Lasers were initially considered ideal for sinus surgery because lasers ablate or vaporize tissue with heat, which also acts to cauterize and seal the small blood vessels in the tissue. Unfortunately, lasers are both expensive and somewhat tedious to use in these procedures. Another disadvantage with lasers is the difficulty in judging the depth of tissue ablation. Since the surgeon generally points and shoots the laser without contacting the tissue, he or she does not receive any tactile feedback to judge how deeply the laser is cutting. Because healthy tissue, cartilage, bone and/or cranial nerves often lie within close proximity of the sinus tissue, it is essential to maintain a minimum depth of tissue damage, which cannot always be ensured with a laser.

Sleep-apnea syndrome is a medical condition characterized by daytime hypersomnolence, intellectual deterioration, cardiac arrhythmias, snoring and thrashing during sleep. This syndrome is classically divided into two types. One type, termed "central sleep apnea syndrome", is characterized by repeated loss of respiratory effort. The second type, termed obstructive sleep apnea syndrome, is characterized by repeated apneic episodes during sleep resulting from obstruction of the patient's upper airway or that portion of the patient's respiratory tract which is cephalad to, and does not include, the larynx.

Treatment for sleep apnea has included various medical, surgical and physical measures. Medical measures include the use of medications and the avoidance of central nervous system depressants, such as sedatives or alcohol. These measures are sometimes helpful, but rarely completely effective. Physical measures have included weight loss, nasopharygeal airways, nasal CPAP and various tongue retaining devices used nocturnally. These measures are cumbersome, uncomfortable and difficult to use for prolonged periods of time. In particular, CPAP devices, which act essentially as a pneumatic "splint" to the airway to alleviate the obstruction, may last for the entire patient's lifetime, and typically requires close to 100% usage of the device while sleeping and napping.

Surgical interventions have included uvulopalatopharyngoplasty (UPPP), laser-assisted uvuloplasty procedures (LAUP), tonsillectomy, surgery to correct severe retrognathia and tracheostomy. The LAUP procedures involve the use a CO₂ laser to excise and vaporize excess tissue in the region of the palate and uvula. In UPPP procedures, a scalpel or conventional electrocautery device is typically employed to remove portions of the uvula, palate, pharynx and/or tonsils. While these procedures are effective, the risk of surgery in some patients is often prohibitive. In addition, UPPP and LAUP procedures performed with conventional electrocautery or laser devices typically generate excessive bleeding and extreme post-operative pain which may be unacceptable to the patient.

Recently, RF energy has been used to selectively destroy portions of the tongue to treat air passage disorders, such as sleep apnea. This procedure, which was developed by Somnus Medical Technologies of Sunnyvale, California, involves the use of a monopolar electrode that directs RF current into the target tissue to desiccate or destroy submucosal tissue in the patient's mouth. Of course, such monopolar devices suffer from the disadvantage that the electric current will flow through undefined paths in the patient's body, thereby increasing the risk of unwanted electrical stimulation to portions of the patient's body. In addition, since the defined path through the patient's body has a relatively high impedance (because of the large distance or resistivity of the patient's body), large voltage differences must typically be applied between the return and active electrodes in order to generate a current suitable for ablation or cutting of the target tissue. This current, however, may inadvertently flow along body paths having less impedance than the defined electrical path, which will substantially increase the current flowing through these paths, possibly causing damage to or destroying surrounding tissue or neighboring peripheral nerves.

Another disadvantage of conventional RF devices, such as the Somnus monopolar electrode, is that these devices typically operate by creating a voltage difference between the active electrode and the target tissue, causing an electrical arc to form across the physical gap between the electrode and tissue. At the point of contact of the electric arcs with tissue, rapid tissue heating occurs due to high current density between the electrode and tissue. This high current density causes cellular fluids to rapidly vaporize into steam, thereby producing a "cutting effect" along the pathway of localized tissue heating. Thus, the tissue is parted along the pathway of evaporated cellular fluid, inducing undesirable collateral tissue damage in regions surrounding the target tissue site. This collateral tissue damage often causes indiscriminate destruction of tissue, resulting in the loss of the proper function of the tissue.

US-A-4,674,499 discloses a plurality of embodiments of bipolar electrode probe devices for use in electrocautery and electrocoagulation.

WO-96/39914 discloses a system for electrosurgical cutting and ablation wherein the fluid outlet is not recessed from an outer surface of the apparatus.

### SUMMARY OF THE INVENTION

According to the present invention, there is provided an apparatus as set out in claim 1.

There are also described herein systems, apparatus and methods for selectively applying electrical energy to structures in the head and neck of a patient's body, such as tissue within the ear, nose and throat. The systems and methods described are particularly useful for ablation and hemostasis of tissue in sinus surgery (e.g., chronic sinusitis and/or removal of polypectomies) and collagen shrinkage, ablation and/or hemostasis in procedures for treating snoring and obstructive sleep apnea (e.g., soft palate or tongue/pharynx stiffening, and midline glossectomies).

One method described herein comprises positioning an electrosurgical probe adjacent the target tissue so that one or more electrode terminal(s) are brought into at least partial contact or close proximity with the target site. Electrically conducting fluid, such as isotonic saline, is directed to the target site to generate a current flow path between the electrode terminal(s) and one or more return electrode(s). High frequency voltage is then applied between the electrode terminal(s) and the return electrode(s) through the current flow path created by the electrically conducting fluid. Depending on the procedure, the probe may then be translated, reciprocated or otherwise manipulated to cut, remove, ablate, contract, coagulate, vaporize, desiccate or otherwise modify the tissue structure at the target site. This method is particularly effective in a naturally dry environment (i.e., the tissue is not submerged in fluid), such as nasal or oral surgery, because the electrically conducting fluid provides a suitable current flow path from the electrode terminal(s) to, the return electrode(s).

There is also described herein, a method for removing tissue in the nasal cavity or a paranasal sinus of a patient to remove a blockage, such as swollen nasal tissue, mucus membranes, turbinates, polyps, neoplasms, cartilage (e.g., the nasal septum) or the like. In this method, one or more electrode terminal(s) are delivered into the nasal cavity, either endoscopically through one of the nasal passages or directly in an open procedure. An electrically conductive fluid, such as isotonic saline, is delivered to the target site within or around the nasal cavity to substantially surround the electrode terminal(s) with the fluid. Alternatively, a more viscous fluid, such as an electrically conductive gel, may be applied to the target site such that the electrode terminal(s) are submerged within the gel during the procedure. In both embodiments, high frequency voltage is applied between the electrode terminal(s) and one or more return electrode(s) to remove at least a portion of the tissue. The high frequency voltage is preferably selected to effect a controlled depth of hemostasis of severed blood vessels within the tissue, which greatly improves the surgeon's view of the surgical site.

In a specific configuration, the nasal tissue is removed by molecular dissociation or disintegration processes. In these embodiments, the high frequency voltage applied to the electrode terminal(s) is sufficient to vaporize an electrically conductive fluid (e.g., gel or saline) between the electrode terminal(s) and the tissue. Within the vaporized fluid, a ionised plasma is formed and charged particles (e.g., electrons) are accelerated towards the tissue to cause the molecular breakdown or disintegration of several cell layers of the tissue. This molecular dissociation is accompanied by the volumetric removal of the tissue. The short range of the accelerated charged particles within the plasma layer confines the molecular dissociation process to the surface layer to minimize damage and necrosis to the underlying tissue. This process can be precisely controlled to effect the volumetric removal of tissue as thin as 10 to 150 microns with minimal heating of, or damage to, surrounding or underlying tissue structures. A more complete description of this phenomena is described in commonly assigned U.S. Patent No. 5,683,366.

The system described offers a number of advantages over current microdebrider and laser techniques for endoscopic sinus surgery. The ability to precisely control the volumetric removal of tissue results in a field of tissue ablation or removal that is very defined, consistent and predictable. The shallow depth of tissue heating also helps to minimize or completely eliminate damage to healthy tissue structures, cartilage, bone and/or cranial nerves that are often adjacent the target sinus tissue. In addition, small blood vessels within the nose are simultaneously cauterized and sealed as the tissue is removed to continuously maintain hemostasis during the procedure. This increases the surgeon's field of view, and shortens the length of the procedure. Moreover, since the system allows for the use of electrically conductive fluid (contrary to prior art bipolar and monopolar electrosurgery techniques), isotonic saline may be used during the procedure. Saline is the preferred medium for irrigation because it has the same concentration as the body's fluids and, therefore, is not absorbed into the body as much as other fluids.

A small tissue segment or hole is removed from the region near or in the turbinates within the nose to shrink the turbinates and prevent swelling, due to the formation of scar tissue as the wound heals. One or more electrode terminals are positioned adjacent the target tissue near the turbinates, and fluid is delivered or applied to the target tissue as described above. As high frequency energy is applied to the electrode terminal(s) and the return electrode(s), the electrode terminals are advanced into the space vacated by the removed tissue to form a small hole or channel in the turbinate tissue. This hole or holes, typically less than 3 mm diameter, preferably less than 1 mm in diameter, help to shrink the rurbinates and prevent swelling. In an exemplary embodiment, an incision is performed (i.e., with a separate instrument, or with the electrosurgical probe of the present invention), so that the mucosa can be lifted before ablating the underlying tissue. This helps to preserve the mucosa and its important function to the nose. Alternatively, the holes may be made directly through mucosa, which should not adversely affect mucosal transport given the small size of the holes formed by the present invention. A more complete description of electrosurgical methods for forming holes or channels in tissue can be found in U.S. Patent No. 5,683,366.

There is also described herein a method for positioning one or more electrode terminal(s) in close proximity to a target site within the head or neck of a patient, such as the nasal cavity or in the patient's throat. High frequency voltage is applied to the electrode terminal(s) to elevate the temperature of collagen fibers within the tissue at the target site from body temperature (about 37 °C) to a tissue temperature in the range of about 45°C to 90°C, usually about 60°C to 70°C, to substantially irreversibly contract these collagen fibers. In a preferred embodiment, an electrically conducting fluid is provided between the electrode terminal(s) and one or more return electrode(s) positioned proximal to the electrode terminal(s) to provide a current flow path from the electrode terminal(s) away from the tissue to the return electrode (s). The current flow path may be generated by directing an electrically conducting fluid along a fluid path past the return electrode and to the target site, or by locating a viscous electrically conducting fluid, such as a gel, at the target site, and submersing the electrode terminal(s) and the return electrode(s) within the conductive gel. The collagen fibers may be heated either by passing the electric current through the tissue to a selected depth before the current returns to the return electrode(s) and/or by heating the electrically conducting fluid and generating a jet or plume of heated fluid, which is directed towards the target tissue. In the latter embodiment, the electric current may not pass into the tissue at all. In both embodiments, the heated fluid and/or the electric current elevates the temperature of the collagen sufficiently to cause hydrothermal shrinkage of the collagen fibers.

The contraction of collagen tissue is particularly useful in procedures for treating obstructive sleep disorders, such as snoring or sleep apnea. In these procedures, one or more electrode terminal(s) are introduced into the patient's mouth, and positioned adjacent the target tissue, selected portions of the tongue, tonsils, soft palate tissues (e.g., the uvula), hard tissue and mucosal tissue. An endoscope or other type of viewing device, may also be introduced, or partially introduced, into the mouth to allow the surgeon to view the procedure (the viewing device may be integral with, or separate from, the electrosurgical probe). Electrically conductive fluid is applied as described above, and high frequency voltage is applied to the electrode terminal(s) and one or more return electrode(s) to, for example, ablate or shrink sections of the uvula without causing unwanted tissue damage under and around the selected sections of tissue.

Apparatus such as those described herein generally include an electrosurgical probe or handpiece having a shaft with proximal and distal ends, one or more electrode terminal(s) at the distal end and one or more connectors coupling the electrode terminal(s) to a source of high frequency electrical energy. For endoscopic sinus surgery, the shaft will have at least a distal end portion sized for delivery through one of the patient's nasal passages. For treating swollen turbinates, the distal end portion of the shaft will usually have a diameter of less than 3 mm, preferably less than 1 mm, to facilitate the formation of small hole(s) or channel(s) within the swollen turbinate tissue. The shaft may additionally include a lens at the distal end coupled to a proximal eye piece for endoscopically viewing the target tissue. Alternatively, the endoscope may be a separate instrument that is introduced through the same or a different opening as the electrosurgical probe.

The apparatus will preferably further include a fluid delivery element for delivering electrically conducting fluid to the electrode terminal(s) and the target site. The fluid delivery element may be located on the probe, e.g., a fluid lumen or tube, or it may be part of a separate instrument. Alternatively, an electrically conducting gel or spray, such as a saline electrolyte or other conductive gel, may be applied the target site. In this embodiment, the apparatus may not have a fluid delivery element. In both embodiments, the electrically conducting fluid will preferably generate a current flow path between the electrode terminal(s) and one or more return electrode(s). In an exemplary embodiment, the return electrode is located on the probe and spaced a sufficient distance from the electrode terminal(s) to substantially avoid or minimize current shorting therebetween and to shield the return electrode from tissue at the target site.

In a specific configuration, the electrosurgical probe will include an electrically insulating electrode support member having a tissue treatment surface at the distal end of the probe. One or more electrode terminal(s) are coupled to, or integral with, the electrode support member such that the electrode terminal(s) are spaced from the return electrode. In one embodiment, the probe includes an electrode array having a plurality of electrically isolated electrode terminals embedded into the electrode support member such that the electrode terminals extend about 0.2 mm to about 10 mm distally from the tissue treatment surface of the electrode support member. In this embodiment, the probe will further include one or more lumens for delivering electrically conductive fluid to one or more openings around the tissue treatment surface of the electrode support member. In an exemplary embodiment, the lumen will extend through a fluid tube exterior to the probe shaft that ends proximal to the return electrode.

The system may optionally include a temperature controller coupled to one or more temperature sensors at or near the distal end of the probe. The controller adjusts the output voltage of the power supply in response to a temperature set point and the measured temperature value. The temperature sensor may be, for example, a thermocouple, located in the insulating support that measures a temperature at the distal end of the probe. In this embodiment, the temperature set point will preferably be one that corresponds to a tissue temperature that results, for example, in the contraction of the collagen tissue, i.e., about 60°C to 70°C. Alternatively, the temperature sensor may directly measure the tissue temperature (e.g., infrared sensor).

There is also described herein a method for removing occlusive media in one of the small body passages coupled to the nasal cavity or a paranasal sinus of a patient to remove a blockage, such as swollen nasal tissue, mucus membranes, polyps, neoplasms, or the like. In this method, one or more electrode terminal(s) are delivered into the nasal cavity, either endoscopically through one of the nasal passages or directly in an open procedure. An electrically conductive fluid, such as isotonic saline, is delivered to the target site within or around the nasal cavity to substantially surround the electrode terminal(s) with the fluid. The fluid may be delivered through an instrument to the specific target site, or the entire nasal cavity may be filled with conductive fluid such that the electrode terminal(s) are submerged during the procedure. In both embodiments, high frequency voltage is applied between the electrode terminal(s) and one or more return electrode(s) to volumetrically remove or ablate at least a portion of the occlusive media within the body passage.

The apparatus will generally include an electrosurgical catheter having a shaft with proximal and distal ends, one or more electrode terminal(s) at the distal end and one or more connectors coupling the electrode terminal(s) to a source of high frequency electrical energy. The catheter shaft will have at least a distal end portion sized for delivery through the small body lumens coupled to the nasal cavity and/or the sinus cavities. Depending on the patient's body structure, the distal end portion of the shaft will usually have a diameter of less than 3 mm, preferably less than 0.5 mm. The shaft may additionally include a lens at the distal end coupled to a proximal eye piece for endoscopically viewing the target tissue. Alternatively, the endoscope may be a separate instrument that is introduced through the same or a different opening as the electrosurgical catheter.

There are also described herein, systems and methods for ablation and hemostasis of swollen or enlarged tissue structures in the nose, such as turbinates. The method described comprises positioning an electrosurgical instrument adjacent an enlarged body structure so that one or more electrode terminal(s) are brought into at least partial contact or close proximity with the body structure. High frequency voltage is applied between the electrode terminal(s) and one or more return electrode(s) to volumetrically remove at least a portion of the body structure. The electrode terminal(s) may be translated relative to the body structure during or after the application of electrical energy to sculpt a void within the body structure, such as a hole, channel, stripe, crater, or the like. The electrode terminal(s) are axially translated toward the body structure to bore one or more channel(s) or hole(s) through a portion of the structure. The electrode terminal(s) are translated across the body structure to form one or more stripe(s) or channel(s). Electrically conducting fluid, such as isotonic saline, is located between the electrode terminal(s) and the body structure. In the bipolar modality, the conducting fluid generates a current flow path between the electrode terminal(s) and one or more return electrode(s). High frequency voltage is then applied between the electrode terminal(s) and the return electrode(s) through the current flow path created by the electrically conducting fluid.

There is also described herein, a method for reducing the volume of enlarged swollen tissue in the patient's nose, such as swollen nasal tissue, mucus membranes, turbinates, polyps, neoplasms or the like. In particular, a turbinate is treated by positioning one or more electrode terminal(s) adjacent to the turbinate, and delivering electrically conductive fluid, such as isotonic saline, to the nasal cavity to substantially surround the electrode terminal(s) with the fluid. Alternatively, a more viscous fluid, such as an electrically conductive gel, may be applied to the target site such that the electrode terminal(s) are submerged within the gel during the procedure. In both embodiments, high frequency voltage is applied between the electrode terminal(s) and one or more return electrode(s) to remove a small tissue segment, channel or hole from the region near or in the turbinates to shrink the turbinates and prevent swelling, due to the formation of scar tissue as the wound heals. The high frequency voltage may be selected to effect a small amount of thermal damage to the walls of the channel or hole to facilitate the formation of scar tissue without extending this thermal damage beyond the immediate region of the target site.

The hole(s) or channel(s) formed by the systems described, typically less than 3 mm diameter, preferably less than 1 mm in diameter, help to shrink the turbinates and prevent swelling. An incision is performed (i.e., with a separate instrument, or with the electrosurgical probe of the present invention), so that the mucosa can be lifted before ablating the underlying tissue. This helps to preserve the mucosa and its important function to the nose. Alternatively, the holes may be made directly through mucosa, which should not adversely affect mucosal transport given the small size of the holes formed by the present invention. A more complete description of electrosurgical methods for forming holes or channels in tissue can be found in U.S. Patent No. 5,683,366.

In another embodiment, an electrosurgical instrument, such as a probe or catheter, comprises a shaft with proximal and distal ends, one or more electrode terminal(s) at the distal end and one or more connectors coupling the electrode terminal(s) to a source of high frequency electrical energy. In this embodiment, the electrode terminal(s) are preferably designed for cutting tissue; i.e., they typically have a distal edge or point. Conventional electrosurgery cuts through tissue by rapidly heating the tissue until cellular fluids explode, producing a cutting effect along the pathway of localized heating. The present system volumetrically removes the tissue along the cutting pathway in a cool ablation process that minimizes thermal damage to surrounding tissue.. The electrode terminal(s) are preferably designed for cutting tissue; i.e., they typically have a distal edge or point. In the exemplary embodiment, the electrode terminal(s) are aligned with each other to form a linear cutting path through the tissue.

In yet another aspect of the systems described, systems and methods are provided for ablation, cutting and hemostasis of tissue for gross tissue removal in the mouth and throat, and for treating obstructive sleep disorders, such as snoring or sleep apnea. The present system removes obstructive tissue within the mouth and throat with a novel, precise process that involves lower temperatures than conventional RF devices and lasers used for such tissue removal. Consequently, the gross tissue removal of the present invention provides significantly less collateral tissue damage, which results in less post-operative pain and faster healing than conventional procedures.

Methods described herein include introducing one or more electrode terminal(s) into the patient's mouth, and positioning the electrode terminal(s) adjacent the target tissue, e.g., selected portions of the tongue, tonsils, soft palate tissues (e.g., the uvula and pharynx), hard tissue or other mucosal tissue. Electrically conductive fluid, such as isotonic saline, is delivered to the target site within the mouth to substantially surround the electrode terminal(s) with the fluid. The fluid may be delivered through an instrument to the specific target site, or the entire target region may be filled with conductive fluid such that the electrode terminal(s) are submerged during the procedure. In both embodiments, high frequency voltage is applied between the electrode terminal(s) and one or more return electrode(s) to volumetrically remove or ablate at least a portion of the obstructive tissue *in situ*. The high frequency voltage may also be selected to effect a controlled depth of hemostasis of severed blood vessels within the tissue, which greatly improves the surgeon's view of the surgical site during the procedure.

### BRIEF DESCRIPTION OF THE DRAWINGS

Fig. 1 is a perspective view of an electrosurgical system incorporating a power supply and an electrosurgical probe for tissue ablation, resection, incision, contraction and for vessel hemostasis according to the present invention;
Fig. 2 is a side view of an electrosurgical probe according to the present invention;
Fig. 3 is an end view of the probe of Fig. 2
Fig. 4 is a cross sectional view of the electrosurgical probe of Fig. 1;
Fig. 5 is an exploded view of a proximal portion of the electrosurgical probe;
Figs. 6A and 6B are perspective and end views, respectively, of an alternative electrosurgical probe incorporating an inner fluid lumen;
Figs. 7A-7C are cross-sectional views of the distal portions of three different embodiments of an electrosurgical probe; which is helpful to understand the present invention;
Figs. 8A and 8B are cross-sectional and end views, respectively, of yet another electrosurgical probe incorporating flattened electrode terminals;
Fig. 9 illustrates an electrosurgical probe with a 90° distal bend and a lateral fluid lumen;
Fig. 10 illustrates an electrosurgical system with a separate fluid delivery instrument, which is helpful to understand the present invention,
Fig. 11 illustrates an endoscopic sinus surgery procedure, wherein an endoscope is delivered through a nasal passage to view a surgical site within the nasal cavity of the patient;
Fig. 12 illustrates an endoscopic sinus surgery procedure with one of the probes described above according to the present invention;
Figs. 13A and 13B illustrate a detailed view of the sinus surgery procedure, illustrating ablation of tissue which is helpful to understand the present invention; and
Fig. 14 illustrates a procedure for treating obstructive sleep disorders, such as sleep apnea, according to the present invention.
Fig. 15 illustrates a catheter system for electrosurgical treatment of body structures within the head and neck according to the present invention;
Fig. 16 is a cross-section view of a working end of a catheter which is helpful to understand the present invention;
Fig. 17A is a cross-section view of a working end of a catheter which is helpful to understand the present invention;
Fig. 17B is an end view of the catheter of Fig. 17A;
Fig. 18 is a sagittal section of a patient's head illustrating a method of removing tissue from body lumens within a patient's nose according to the present invention;
Fig. 19 is a coronal section of a patient's head illustrating the paranasal sinuses;
Fig. 20 is a detailed end view of an electrosurgical probe having an elongate, linear array of electrode terminals suitable for use in surgical cutting;
Fig. 21 is a detailed view of a single electrode terminal having a flattened end at its distal tip;
Fig. 22 is a detailed view of a single electrode terminal having a pointed end at its distal tip;
Fig. 23 is a perspective view of another embodiment of an electrosurgical probe for use in dermatology procedures;
Fig. 24 is an end view of the distal tip of the probe, illustrating an electrode support with a plurality of electrode terminals;
Fig. 25 illustrates the electrical connections and the electrode support of the handpiece in greater detail;
Fig. 26 is a perspective view of the distal portion of another electrosurgical probe which is helpful to understand the present invention;
Figs. 27 and 28 are partial cross-sectional views of a distal portion of electrosurgical probes designed for cutting and coagulation of tissue;
Fig. 29 illustrates a procedure for treating turbinates with one of the above probes; and
Figs. 30A and 30B illustrate a detailed view of the turbinate procedure of Fig. 29, illustrating ablation of tissue which is helpful to understand the present invention.

### DESCRIPTION OF SPECIFIC EMBODIMENTS

The present invention provides systems for selectively applying electrical energy to a target location within or on a patient's body, particularly including tissue in head and neck, such as the ear, mouth, pharynx, larynx, esophagus, nasal cavity and sinuses. These procedures may be performed through the mouth or nose using speculae or gags, or using endoscopic techniques, such as functional endoscopic sinus surgery (FESS). These procedures may include the removal of swollen tissue, chronically-diseased inflamed and hypertrophic mucus linings, polyps and/or neoplasms from the various anatomical sinuses of the skull, the turbinates and nasal passages, in the tonsil, adenoid, epi-glottic and supra-glottic regions, and salivary glands, submucus resection of the nasal septum, excision of diseased tissue and the like. In other procedures, the present invention may be useful for collagen shrinkage, ablation and/or hemostasis in procedures for treating snoring and obstructive sleep apnea (e.g., soft palate, such as the uvula, or tongue/pharynx stiffening, and midline glossectomies), for gross tissue removal, such as tonsillectomies, adenoidectomies, tracheal stenosis and vocal cord polyps and lesions, or for the resection or ablation of facial tumors or tumors within the mouth and pharynx, such as glossectomies, laryngectomies, acoustic neuroma procedures and nasal ablation procedures. In addition, the present invention is useful for procedures within the ear, such as stapedotomies, tympanostomies or the like.

The present invention may also be useful for cosmetic and plastic surgery procedures in the head and neck. For example, the present invention is particularly useful for ablation and sculpting of cartilage tissue, such as the cartilage within the nose that is sculpted during rhinoplasty procedures. The present invention may also be employed for skin tissue removal and/or collagen shrinkage in the epidermis or dermis tissue in the head and neck, e.g., the removal of pigmentations, vascular lesions (e.g., leg veins), scars, tattoos, etc., and for other surgical procedures on the skin, such as tissue rejuvenation, cosmetic eye procedures (blepharoplasties), wrinkle removal, tightening muscles for facelifts or browlifts, hair removal and/or transplant procedures, etc.

The present invention is useful for cutting, resection, ablation and/or hemostasis of tissue in procedures for treating snoring and obstructive sleep apnea (e.g., UPPP and LAUP procedures), for gross tissue removal, such as tonsillectomies, adenoidectomies, tracheal stenosis and vocal cord polyps and lesions, or for the resection or ablation of facial tumors or tumors within the mouth and pharynx, such as glossectomies, laryngectomies, acoustic neuroma procedures and nasal ablation procedures. For convenience, the remaining disclosure will be directed specifically to the treatment of sleep obstructive disorders, but it will be appreciated that the system and method can be applied equally well to procedures involving other tissues of the body, as well as to other procedures including open procedures, intravascular procedures, urology, laparascopy, arthroscopy, thoracoscopy or other cardiac procedures, cosmetic surgery, orthopedics, gynecology, otorhinolaryngology, spinal and neurologic procedures, oncology and the like.

In some embodiments of the present invention, high frequency (RF) electrical energy is applied to one or more electrode terminals in the presence of electrically conductive fluid to remove and/or modify the structure of tissue structures. Depending on the specific procedure, the present invention may be used to: (1) volumetrically remove tissue, bone or cartilage (i.e., ablate or effect molecular dissociation of the tissue structure); (2) form holes, channels, divots or other spaces within tissue (3) cut or resect tissue; (4) shrink or contract collagen connective tissue; and/or (5) coagulate severed blood vessels.

In one aspect of the invention, the obstructive tissue is volumetrically removed or ablated. In this procedure, a high frequency voltage difference is applied between one or more electrode terminal(s) and one or more return electrode(s) to develop high electric field intensities in the vicinity of the target tissue. The high electric field intensities adjacent the electrode terminal(s) lead to electric field induced molecular breakdown of target tissue through molecular dissociation (rather than thermal evaporation or carbonization). Applicant believes that the tissue structure is volumetrically removed through molecular disintegration of larger organic molecules into smaller molecules and/or atoms, such as hydrogen, oxygen, oxides of carbon, hydrocarbons and nitrogen compounds. This molecular disintegration completely removes the tissue structure, as opposed to dehydrating the tissue material by the removal of liquid within the cells of the tissue, as is typically the case with electrosurgical desiccation and vaporization.

The high electric field intensities may be generated by applying a high frequency voltage that is sufficient to vaporize an electrically conducting fluid over at least a portion of the electrode terminal(s) in the region between the distal tip of the electrode terminal(s) and the target tissue. The electrically conductive fluid may be a liquid, such as isotonic saline or blood, delivered to the target site, or a viscous fluid, such as a gel, applied to the target site. Since the vapor layer or vaporized region has a relatively high electrical impedance, it increases the voltage differential between the electrode terminal tip and the tissue and causes ionization within the vapor layer due to the presence of an ionizable species (e.g., sodium when isotonic saline is the electrically conducting fluid). This ionization, under optimal conditions, induces the discharge of energetic electrons and photons from the vapor layer and to the surface of the target tissue. This energy may be in the form of energetic photons (e.g., ultraviolet radiation), energetic particles (e.g., electrons) or a combination thereof. A more detailed description of this phenomena, termed Coblation^{™} can be found in commonly assigned U.S. Patent No. 5,683,366 the complete disclosure of which is incorporated herein by reference.

In some procedures, e.g., soft palate or tongue/pharynx stiffening, it may be desired to shrink or contract collagen connective tissue at the target site. In these procedures, the RF energy heats the tissue directly by virtue of the electrical current flow therethrough, and/or indirectly through the exposure of the tissue to fluid heated by RF energy, to elevate the tissue temperature from normal body temperatures (e.g., 37°C) to temperatures in the range of 45°C to 90°C, preferably in the range from about 60°C to 70°C. Thermal shrinkage of collagen fibers occurs within a small temperature range which, for mammalian collagen is in the range from 60°C to 70°C (Deak, G., et al., "The Thermal Shrinkage Process of Collagen Fibres as Revealed by Polarization Optical Analysis of Topooptical Staining Reactions," Acta Morphologica Acad. Sci. of Hungary, Vol. 15(2), pp 195-208, 1967). Collagen fibers typically undergo thermal shrinkage in the range of 60°C to about 70°C. Previously reported research has attributed thermal shrinkage of collagen to the cleaving of the internal stabilizing cross-linkages within the collagen matrix (Deak, ibid). It has also been reported that when the collagen temperature is increased above 70°C, the collagen matrix begins to relax again and the shrinkage effect is reversed resulting in no net shrinkage (Allain, J. C., et al., "Isometric Tensions Developed During the Hydrothermal Swelling of Rat Skin," Connective Tissue Research, Vol. 7, pp 127-133, 1980). Consequently, the controlled heating of tissue to a precise depth is critical to the achievement of therapeutic collagen shrinkage. A more detailed description of collagen shrinkage can be found in U.S. Patent Application No. 08/942,580 filed on October 2, 1997, (Attorney Docket No. 16238-001300).

The preferred depth of heating to effect the shrinkage of collagen in the heated region (i.e., the depth to which the tissue is elevated to temperatures between 60°C to 70°C) generally depends on (1) the thickness of the tissue, (2) the location of nearby structures (e.g., nerves) that should not be exposed to damaging temperatures, and/or (3) the location of the collagen tissue layer within which therapeutic shrinkage is to be effected. The depth of heating is usually in the range from 0 to 3.5 mm. In the case of collagen within the soft palate or uvula, the depth of heating is preferably in the range from about 0.5 to about 3.5 mm.

In other embodiments, the present invention applies high frequency (RF) electrical energy in an electrically conducting fluid environment to remove (i.e., resect, cut or ablate) a tissue structure and to seal transected vessels within the region of the target tissue. The present invention is particularly useful for sealing larger arterial vessels, e.g., on the order of 1 mm or greater. In some embodiments, a high frequency power supply is provided having an ablation mode, wherein a first voltage is applied to an electrode terminal sufficient to effect molecular dissociation or disintegration of the tissue, and a coagulation mode, wherein a second, lower voltage is applied to an electrode terminal (either the same or a different electrode) sufficient to achieve hemostasis of severed vessels within the tissue. In other embodiments, an electrosurgical instrument is provided having one or more coagulation electrode(s) configured for sealing a severed vessel, such as an arterial vessel, and one or more electrode terminals configured for either contracting the collagen fibers within the tissue or removing (ablating) the tissue, e.g., by applying sufficient energy to the tissue to effect molecular dissociation. In the latter embodiments, the coagulation electrode(s) may be configured such that a single voltage can be applied to coagulate with the coagulation electrode(s), and to ablate with the electrode terminal(s). In other embodiments, the power supply is combined with the coagulation instrument such that the coagulation electrode is used when the power supply is in the coagulation mode (low voltage), and the electrode terminal(s) are used when the power supply is in the ablation mode (higher voltage).

One or more electrode terminals are brought into close proximity to tissue at a target site, and the power supply is activated in the ablation mode such that sufficient voltage is applied between the electrode terminals and the return electrode to volumetrically remove the tissue through molecular dissociation, as described below. During this process, vessels within the tissue will be severed. Smaller vessels will be automatically sealed with the system and method of the present invention. Larger vessels, and those with a higher flow rate, such as arterial vessels, may not be automatically sealed in the ablation mode. In these cases, the severed vessels may be sealed by activating a control (e.g., a foot pedal) to reduce the voltage of the power supply into the coagulation mode. In this mode, the electrode terminals may be pressed against the severed vessel to provide sealing and/or coagulation of the vessel. Alternatively, a coagulation electrode located on the same or a different instrument may be pressed against the severed vessel. Once the vessel is adequately sealed, the surgeon activates a control (e.g., another foot pedal) to increase the voltage of the power supply back into the ablation mode.

The present invention is also useful for removing or ablating tissue around nerves, such as spinal, or cranial nerves, e.g., the hypoglossal nerve, the optic nerve, facial nerves, vestibulocochlear nerves and the like. This is particularly advantageous when removing tissue that is located close to nerves. One of the significant drawbacks with the prior art RF devices, scalpels and lasers is that these devices do not differentiate between the target tissue and the surrounding nerves or bone. Therefore, the surgeon must be extremely careful during these procedures to avoid damage to the nerves within and around the patient's mouth and throat. In the present invention, the Coblation^{™} process for removing tissue results in extremely small depths of collateral tissue damage as discussed above. This allows the surgeon to remove tissue close to a nerve without causing collateral damage to the nerve fibers.

According to the present invention, systems are provided for distinguishing between the fatty tissue immediately surrounding nerve fibers and the normal tissue that is to be removed during the procedure. Nerves usually comprise a connective tissue sheath, or epineurium, enclosing the bundles of nerve fibers, each bundle being surrounded by its own sheath of connective tissue (the perineurium) to protect these nerve fibers. The outer protective tissue sheath or epineurium typically comprises a fatty tissue (e.g., adipose tissue) having substantially different electrical properties than the normal target tissue, such as the turbinates, polyps, mucus tissue or the like, that are, for example, removed from the nose during sinus procedures. The system of the present invention measures the electrical properties of the tissue at the tip of the probe with one or more electrode terminal(s). These electrical properties may include electrical conductivity at one, several or a range of frequencies (e.g., in the range from 1 kHz to 100 MHz), dielectric constant, capacitance or combinations of these. In this embodiment, an audible signal may be produced when the sensing electrode(s) at the tip of the probe detects the fatty tissue surrounding a nerve, or direct feedback control can be provided to only supply power to the electrode terminal(s) either individually or to the complete array of electrodes, if and when the tissue encountered at the tip or working end of the probe is normal tissue based on the measured electrical properties.

In one embodiment, the current limiting elements (discussed in detail above) are configured such that the electrode terminals will shut down or turn off when the electrical impedance reaches a threshold level. When this threshold level is set to the impedance of the fatty tissue surrounding nerves, the electrode terminals will shut off whenever they come in contact with, or in close proximity to, nerves. Meanwhile, the other electrode terminals, which are in contact with or in close proximity to nasal tissue, will continue to conduct electric current to the return electrode. This selective ablation or removal of lower impedance tissue in combination with the Coblation^{™} mechanism of the present invention allows the surgeon to precisely remove tissue around nerves or bone. Applicant has found that the present invention is capable of volumetrically removing tissue closely adjacent to nerves without impairment the function of the nerves, and without significantly damaging the tissue of the epineurium.

In addition to the above, applicant has discovered that the Coblation^{™} mechanism of the present invention can be manipulated to ablate or remove certain tissue structures, while having little effect on other tissue structures. As discussed above, the present invention uses a technique of vaporizing electrically conductive fluid to form a plasma layer or pocket around the electrode terminal(s), and then inducing the discharge of energy from this plasma or vapor layer to break the molecular bonds of the tissue structure. Based on initial experiments, applicants believe that the free electrons within the ionized vapor layer are accelerated in the high electric fields near the electrode tip(s). When the density of the vapor layer (or within a bubble formed in the electrically conducting liquid) becomes sufficiently low (i.e., less than approximately 10²⁰ atoms/cm³ for aqueous solutions), the electron mean free path increases to enable subsequently injected electrons to cause impact ionization within these regions of low density (i.e., vapor layers or bubbles). Energy evolved by the energetic electrons (e.g., 4 to 5 eV) can subsequently bombard a molecule and break its bonds, dissociating a molecule into free radicals, which then combine into final gaseous or liquid species.

The energy evolved by the energetic electrons may be varied by adjusting a variety of factors, such as: the number of electrode terminals; electrode size and spacing; electrode surface area; asperities and sharp edges on the electrode surfaces; electrode materials; applied voltage and power; current limiting means, such as inductors; electrical conductivity of the fluid in contact with the electrodes; density of the fluid; and other factors. Accordingly, these factors can be manipulated to control the energy level of the excited electrons. Since different tissue structures have different molecular bonds, the present invention can be configured to break the molecular bonds of certain tissue, while having too low an energy to break the molecular bonds of other tissue. For example, fatty tissue, (e.g., adipose) tissue has double bonds that require a substantially higher energy level than 4 to 5 eV to break. Accordingly, the present invention in its current configuration generally does not ablate or remove such fatty tissue. Of course, factors may be changed such that these double bonds can also be broken in a similar fashion as the single bonds (e.g., increasing voltage or changing the electrode configuration to increase the current density at the electrode tips). A more complete description of this phenomena can be found in co-pending U.S. Patent Application 09/032,375, filed February 27, 1998 (Attorney Docket No. CB-3).

The present invention also provides apparatus for selectively removing tumors or other undesirable body structures while minimizing the spread of viable cells from the tumor. Conventional techniques for removing such tumors generally result in the production of smoke in the surgical setting, termed an electrosurgical or laser plume, which can spread intact, viable bacterial or viral particles from the tumor or lesion to the surgical team or to other portions of the patient's body. This potential spread of viable cells or particles has resulted in increased concerns over the proliferation of certain debilitating and fatal diseases, such as hepatitis, herpes, HIV and papillomavirus. In the present invention, high frequency voltage is applied between the electrode terminal(s) and one or more return electrode(s) to volumetrically remove at least a portion of the tissue cells in the tumor through the dissociation or disintegration of organic molecules into non-viable atoms and molecules. Specifically, the present invention converts the solid tissue cells into non-condensable gases that are no longer intact or viable, and thus, not capable of spreading viable tumor particles to other portions of the patient's brain or to the surgical staff. The high frequency voltage is preferably selected to effect controlled removal of these tissue cells while minimizing substantial tissue necrosis to surrounding or underlying tissue. A more complete description of this phenomena can be found in co-pending U.S. Patent Application 09/109,219, filed June 30, 1998 (Attorney Docket No. CB-1).

The electrosurgical instrument will comprise a shaft or a handpiece having a proximal end and a distal end which supports one or more electrode terminal(s). The shaft or handpiece may assume a wide variety of configurations, with the primary purpose being to mechanically support the active electrode and permit the treating physician to manipulate the electrode from a proximal end of the shaft. The shaft may be rigid or flexible, with flexible shafts optionally being combined with a generally rigid external tube for mechanical support. Flexible shafts may be combined with pull wires, shape memory actuators, and other known mechanisms for effecting selective deflection of the distal end of the shaft to facilitate positioning of the electrode array. The shaft will usually include a plurality of wires or other conductive elements running axially therethrough to permit connection of the electrode array to a connector at the proximal end of the shaft.

For procedures within the nose, the shaft will have a suitable diameter and length to allow the surgeon to reach the target site (e.g., a blockage in the nasal cavity or one of the sinuses) by delivering the probe shaft through one of the nasal passages or another opening (e.g., an opening in the eye or through an opening surgically creating during the procedure). Thus, the shaft will usually have a length in the range of about 5-25 cm, and a diameter in the range of about 0.5 to 5 mm. For procedures in the small passages of the nose, the shaft diameter will usually be less than 3 mm, preferably less than about 0.5 mm. For procedures requiring the formation of a small hole or channel in tissue, such as treating swollen turbinates, the shaft diameter will usually be less than 3 mm, preferably less than about 1 mm. Likewise, for procedures in the ear, the shaft should have a length in the range of about 3 to 20 cm, and a diameter of about 0.3 to 5 mm. Likewise, for procedures in the ear, the shaft should have a length in the range of about 3 to 20 cm, and a diameter of about 0.3 to 5 mm. For procedures in the mouth or upper throat, the shaft will have any suitable length and diameter that would facilitate handling by the surgeon. For procedures in the lower throat, such as laryngectomies, the shaft will be suitably designed to access the larynx. For example, the shaft may be flexible, or have a distal bend to accommodate the bend in the patient's throat. In this regard, the shaft may be a rigid shaft having a specifically designed bend to correspond with the geometry of the mouth and throat, or it may have a flexible distal end, or it may be part of a catheter. In any of these embodiments, the shaft may also be introduced through rigid or flexible endoscopes. Specific shaft designs will be described in detail in connection with the figures hereinafter.

The electrode terminal(s) are preferably supported within or by an inorganic insulating support positioned near the distal end of the instrument shaft. The return electrode may be located on the instrument shaft, on another instrument or on the external surface of the patient (i.e., a dispersive pad). The close proximity of nerves and other sensitive tissue in the mouth and throat, however, makes a bipolar design more preferable because this minimizes the current flow through non-target tissue and surrounding nerves. Accordingly, the return electrode is preferably either integrated with the instrument body, or another instrument located in close proximity thereto. The proximal end of the instrument(s) will include the appropriate electrical connections for coupling the return electrode(s) and the electrode terminal(s) to a high frequency power supply, such as an electrosurgical generator.

The current flow path between the electrode terminal(s) and the return electrode(s) may be generated by submerging the tissue site in an electrical conducting fluid (e.g., within a viscous fluid, such as an electrically conductive gel) or by directing an electrically conducting fluid along a fluid path to the target site (i.e., a liquid, such as isotonic saline, or a gas, such as argon). The conductive gel may also be delivered to the target site to achieve a slower, more controlled delivery rate of conductive fluid. In addition, the viscous nature of the gel may allow the surgeon to more easily contain the gel around the target site (e.g., rather than attempting to contain isotonic saline). A more complete description of an exemplary method of directing electrically conducting fluid between the active and return electrodes is described in U.S. Patent No. 5,697,281. Alternatively, the body's natural conductive fluids, such as blood, may be sufficient to establish a conductive path between the return electrode(s) and the electrode terminal(s), and to provide the conditions for establishing a vapor layer, as described above. However, conductive fluid that is introduced into the patient is generally preferred over blood because blood will tend to coagulate at certain temperatures. Advantageously, a liquid electrically conductive fluid (e.g., isotonic saline) may be used to concurrently "bathe" the target tissue surface to provide an additional means for removing any tissue, and to cool the region of the target tissue ablated in the previous moment.

The power supply may include a fluid interlock for interrupting power to the electrode terminal(s) when there is insufficient conductive fluid around the electrode terminal(s). This ensures that the instrument will not be activated when conductive fluid is not present, minimizing the tissue damage that may otherwise occur. A more complete description of such a fluid interlock can be found in commonly assigned, co-pending U.S. Application NO. 09/058,336, filed April 10, 1998 (attorney Docket No. CB-4).

In some procedures, it may also be necessary to retrieve or aspirate the electrically conductive fluid and/or the non-condensable gaseous products of ablation. In addition, it may be desirable to aspirate small pieces of tissue or other body structures that are not completely disintegrated by the high frequency energy, or other fluids at the target site, such as blood, mucus, the gaseous products of ablation, etc. Accordingly, the system of the present invention may include one or more suction lumen(s) in the instrument, or on another instrument, coupled to a suitable vacuum source for aspirating fluids from the target site. In addition, the invention may include one or more aspiration electrode(s) coupled to the distal end of the suction lumen for ablating, or at least reducing the volume of, non-ablated tissue fragments that are aspirated into the lumen. The aspiration electrode(s) function mainly to inhibit clogging of the lumen that may otherwise occur as larger tissue fragments are drawn therein. The aspiration electrode(s) may be different from the ablation electrode terminal(s), or the same electrode(s) may serve both functions. A more complete description of instruments incorporating aspiration electrode(s) can be found in commonly assigned, co-pending patent application entitled "Systems And Methods For Tissue Resection, Ablation And Aspiration", filed January 21, 1998.

As an alternative or in addition to suction, it may be desirable to contain the excess electrically conductive fluid, tissue fragments and/or gaseous products of ablation at or near the target site with a containment apparatus, such as a basket, retractable sheath or the like. This embodiment has the advantage of ensuring that the conductive fluid, tissue fragments or ablation products do not flow through the patient's vasculature or into other portions of the body. In addition, it may be desirable to limit the amount of suction to limit the undesirable effect suction may have on hemostasis of severed blood vessels.

The present invention may use a single active electrode terminal or an array of electrode terminals spaced around the distal surface of a catheter or probe. In the latter embodiment, the electrode array usually includes a plurality of independently current-limited and/or power-controlled electrode terminals to apply electrical energy selectively to the target tissue while limiting the unwanted application of electrical energy to the surrounding tissue and environment resulting from power dissipation into surrounding electrically conductive fluids, such as blood, normal saline, and the like. The electrode terminals may be independently current-limited by isolating the terminals from each other and connecting each terminal to a separate power source that is isolated from the other electrode terminals. Alternatively, the electrode terminals may be connected to each other at either the proximal or distal ends of the catheter to form a single wire that couples to a power source.

In one configuration, each individual electrode terminal in the electrode array is electrically insulated from all other electrode terminals in the array within said instrument and is connected to a power source which is isolated from each of the other electrode terminals in the array or to circuitry which limits or interrupts current flow to the electrode terminal when low resistivity material (e.g., blood, electrically conductive saline irrigant or electrically conductive gel) causes a lower impedance path between the return electrode and the individual electrode terminal. The isolated power sources for each individual electrode terminal may be separate power supply circuits having internal impedance characteristics which limit power to the associated electrode terminal when a low impedance return path is encountered. By way of example, the isolated power source may be a user selectable constant current source. In this embodiment, lower impedance paths will automatically result in lower resistive heating levels since the heating is proportional to the square of the operating current times the impedance. Alternatively, a single power source may be connected to each of the electrode terminals through independently actuatable switches, or by independent current limiting elements, such as inductors, capacitors, resistors and/or combinations thereof. The current limiting elements may be provided in the instrument, connectors, cable, controller or along the conductive path from the controller to the distal tip of the instrument. Alternatively, the resistance and/or capacitance may occur on the surface of the active electrode terminal(s) due to oxide layers which form selected electrode terminals (e.g., titanium or a resistive coating on the surface of metal, such as platinum).

The tip region of the instrument may comprise many independent electrode terminals designed to deliver electrical energy in the vicinity of the tip. The selective application of electrical energy to the conductive fluid is achieved by connecting each individual electrode terminal and the return electrode to a power source having independently controlled or current limited channels. The return electrode(s) may comprise a single tubular member of conductive material proximal to the electrode array at the tip which also serves as a conduit for the supply of the electrically conducting fluid between the active and return electrodes. Alternatively, the instrument may comprise an array of return electrodes at the distal tip of the instrument (together with the active electrodes) to maintain the electric current at the tip. The application of high frequency voltage between the return electrode(s) and the electrode array results in the generation of high electric field intensities at the distal tips of the electrode terminals with conduction of high frequency current from each individual electrode terminal to the return electrode. The current flow from each individual electrode terminal to the return electrode(s) is controlled by either active or passive means, or a combination thereof, to deliver electrical energy to the surrounding conductive fluid while minimizing energy delivery to surrounding (non-target) tissue.

The application of a high frequency voltage between the return electrode(s) and the electrode terminal(s) for appropriate time intervals effects cutting, removing, ablating, shaping, contracting or otherwise modifying the target tissue. The tissue volume over which energy is dissipated (i.e., a high current density exists) may be precisely controlled, for example, by the use of a multiplicity of small electrode terminals whose effective diameters or principal dimensions range from about 10 mm to 0.01 mm, preferably from about 2 mm to 0.05 mm, and more preferably from about 1 mm to 0.1 mm. Electrode areas for both circular and non-circular terminals will have a contact area (per electrode terminal) below 50 mm² for electrode arrays and as large as 75 mm² for single electrode embodiments, preferably being in the range from 0.0001 mm² to 1 mm², and more preferably from 0.005 mm² to .5 mm². The circumscribed area of the electrode array is in the range from 0.25 mm² to 75 mm², preferably from 0.5 mm² to 40 mm², and will usually include at least one electrode terminal and in some embodiments includes at least two isolated electrode terminals, often at least five electrode terminals, often greater than 10 electrode terminals and even 50 or more electrode terminals, disposed over the distal contact surfaces on the shaft. The use of small diameter electrode terminals increases the electric field intensity and reduces the extent or depth of tissue heating as a consequence of the divergence of current flux lines which emanate from the exposed surface of each electrode terminal.

The area of the tissue treatment surface can vary widely, and the tissue treatment surface can assume a variety of geometries, with particular areas and geometries being selected for specific applications. The active electrode surface(s) can have area(s) in the range from 0.25 mm² to 75 mm², usually being from about 0.5 mm² to 40 mm². The geometries can be planar, concave, convex, hemispherical, conical, linear "in-line" array or virtually any other regular or irregular shape. Most commonly, the active electrode(s) or electrode terminal(s) will be formed at the distal tip of the electrosurgical instrument shaft, frequently being planar, disk-shaped, or hemispherical surfaces for use in reshaping procedures or being linear arrays for use in cutting. Alternatively or additionally, the active electrode(s) may be formed on lateral surfaces of the electrosurgical instrument shaft (e.g., in the manner of a spatula), facilitating access to certain body structures in endoscopic procedures.

In some embodiments, the electrode support and the fluid outlet may be recessed from an outer surface of the instrument or handpiece to confine the electrically conductive fluid to the region immediately surrounding the electrode support. In addition, the shaft may be shaped so as to form a cavity around the electrode support and the fluid outlet. This helps to assure that the electrically conductive fluid will remain in contact with the electrode terminal(s) and the return electrode(s) to maintain the conductive path therebetween. In addition, this will help to maintain a vapor layer and subsequent plasma layer between the electrode terminal(s) and the tissue at the treatment site throughout the procedure, which reduces the thermal damage that might otherwise occur if the vapor layer were extinguished due to a lack of conductive fluid. Provision of the electrically conductive fluid around the target site also helps to maintain the tissue temperature at desired levels.

The electrically conducting fluid should have a threshold conductivity to provide a suitable conductive path between the return electrode and the electrode terminal(s). The electrical conductivity of the fluid (in units of milliSiemans per centimeter or mS/cm) will usually be greater than 0.2 mS/cm, preferably will be greater than 2 mS/cm and more preferably greater than 10 mS/cm. In an exemplary embodiment, the electrically conductive fluid is isotonic saline, which has a conductivity of about 17 mS/cm.

The voltage difference applied between the return electrode(s) and the electrode terminal(s) will be at high or radio frequency, typically between about 5 kHz and 20 MHz, usually being between about 30 kHz and 2.5 MHz, preferably being between about 50 kHz and 500 kHz, often less than 350 kHz, and often between about 100 kHz and 200 kHz. The RMS (root mean square) voltage applied will usually be in the range from about 5 volts to 1000 volts, preferably being in the range from about 10 volts to 500 volts depending on the electrode terminal size, the operating frequency and the operation mode of the particular procedure or desired effect on the tissue (i.e., contraction, coagulation, cutting or ablation). Typically, the peak-to-peak voltage for ablation or cutting will be in the range of 10 to 2000 volts and preferably in the range of 200 to 1800 volts and more preferably in the range of about 300 to 1500 volts, often in the range of about 500 to 900 volts peak to peak (again, depending on the electrode size, the operating frequency and the operation mode). Lower peak-to-peak voltages will be used for tissue coagulation or collagen contraction and will typically be in the range from 50 to 1500, preferably 100 to 1000 and more preferably 120 to 600 volts peak-to-peak.

As discussed above, the voltage is usually delivered in a series of voltage pulses or alternating current of time varying voltage amplitude with a sufficiently high frequency (e.g., on the order of 5 kHz to 20 MHz) such that the voltage is effectively applied continuously (as compared with e.g., lasers claiming small depths of necrosis, which are generally pulsed about 10 to 20 Hz). In addition, the duty cycle (i.e., cumulative time in any one-second interval that energy is applied) is on the order of about 50 % for the present invention, as compared with pulsed lasers which typically have a duty cycle of about 0.0001 %.

The preferred power source of the present invention delivers a high frequency current selectable to generate average power levels ranging from several milliwatts to tens of watts per electrode, depending on the volume of target tissue being heated, and/or the maximum allowed temperature selected for the instrument tip. The power source allows the user to select the voltage level according to the specific requirements of a particular neurosurgery procedure, cardiac surgery, arthroscopic surgery, dermatological procedure, ophthalmic procedures, open surgery or other endoscopic surgery procedure. For cardiac procedures and potentially for neurosurgery, the power source may have an additional filter, for filtering leakage voltages at frequencies below 100 kHz, particularly voltages around 60 kHz. Alternatively, a power source having a higher operating frequency, e.g., 300 to 500 kHz may be used in certain procedures in which stray low frequency currents may be problematic. A description of one suitable power source can be found in co-pending Patent Applications 09/058,571 and 09/058,336, filed April 10, 1998 (Attorney Docket Nos. CB-2 and CB-4).

The power source may be current limited or otherwise controlled so that undesired heating of the target tissue or surrounding (non-target) tissue does not occur. In a presently preferred embodiment of the present invention, current limiting inductors are placed in series with each independent electrode terminal, where the inductance of the inductor is in the range of 10uH to 50,000uH, depending on the electrical properties of the target tissue, the desired tissue heating rate and the operating frequency. Alternatively, capacitor-inductor (LC) circuit structures may be employed, as described previously in U.S. Patent No. 5,697,909. Additionally, current limiting resistors may be selected. Preferably, these resistors will have a large positive temperature coefficient of resistance so that, as the current level begins to rise for any individual electrode terminal in contact with a low resistance medium (e.g., saline irrigant or blood), the resistance of the current limiting resistor increases significantly, thereby minimizing the power delivery from said electrode terminal into the low resistance medium (e.g., saline irrigant or blood).

It should be clearly understood that the invention is not limited to electrically isolated electrode terminals, or even to a plurality of electrode terminals. For example, the array of active electrode terminals may be connected to a single lead that extends through the catheter shaft to a power source of high frequency current. Alternatively, the instrument may incorporate a single electrode that extends directly through the catheter shaft or is connected to a single lead that extends to the power source. The active electrode(s) may have ball shapes (e.g., for tissue vaporization and desiccation), twizzle shapes (for vaporization and needle-like cutting), spring shapes (for rapid tissue debulking and desiccation), twisted metal shapes, annular or solid tube shapes or the like. Alternatively, the electrode(s) may comprise a plurality of filaments, rigid or flexible brush electrode(s) (for debulking a tumor, such as a fibroid, bladder tumor or a prostate adenoma), side-effect brush electrode(s) on a lateral surface of the shaft, coiled electrode(s) or the like.

In one embodiment, an electrosurgical catheter or probe comprises a single active electrode terminal that extends from an insulating member, e.g., ceramic, at the distal end of the shaft. The insulating member is preferably a tubular structure that separates the active electrode terminal from a tubular or annular return electrode positioned proximal to the insulating member and the active electrode. In another embodiment, the catheter or probe includes a single active electrode that can be rotated relative to the rest of the catheter body, or the entire catheter may be rotated related to the lead. The single active electrode can be positioned adjacent the abnormal tissue and energized and rotated as appropriate to remove this tissue.

The current flow path between the electrode terminal(s) and the return electrode(s) may be generated by submerging the tissue site in an electrical conducting fluid (e.g., within a viscous fluid, such as an electrically conductive gel) or by directing an electrically conducting fluid along a fluid path to the target site (i.e., a liquid, such as isotonic saline, or a gas, such as argon). This latter method is particularly effective in a dry environment (i.e., the tissue is not submerged in fluid) because the electrically conducting fluid provides a suitable current flow path from the electrode terminal to the return electrode.

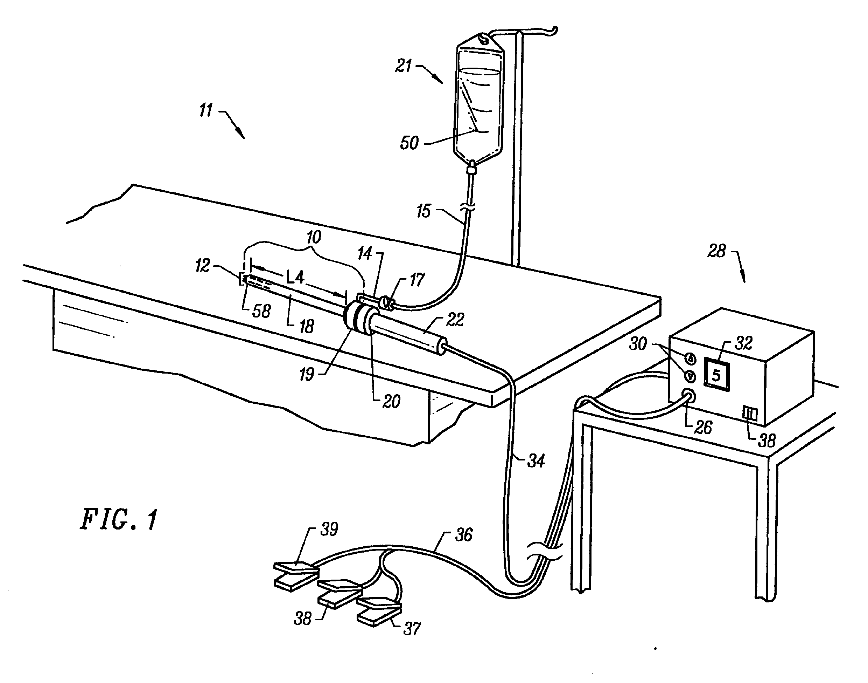

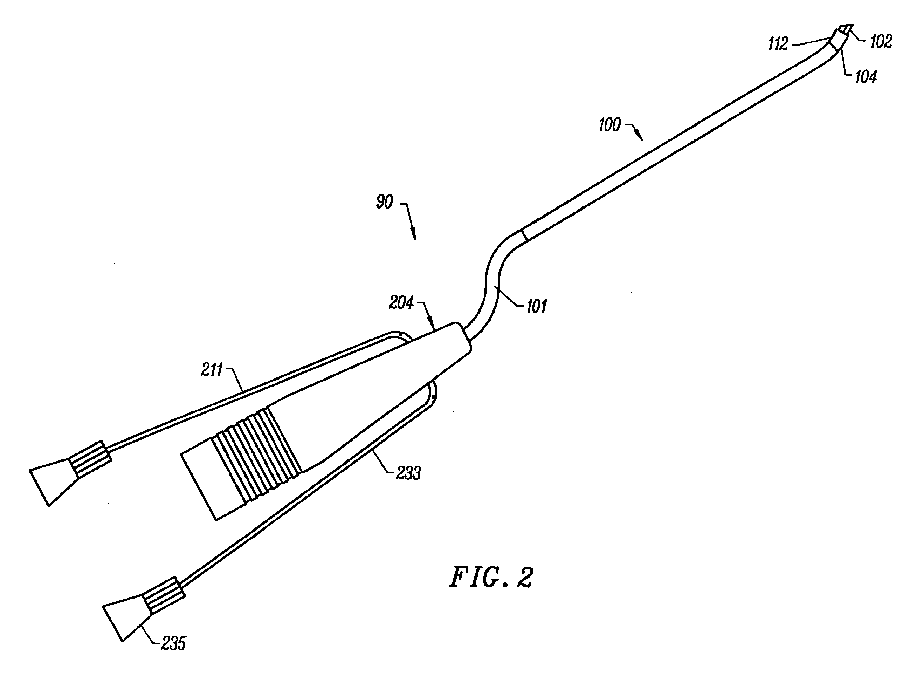

Referring to Fig. 1, an exemplary electrosurgical system 11 for treatment of tissue in the head and neck will now be described in detail. Electrosurgical system 11 generally comprises an electrosurgical handpiece or probe 10 connected to a power supply 28 for providing high frequency voltage to a target site and a fluid source 21 for supplying electrically conducting fluid 50 to probe 10. In addition, electrosurgical system 11 may include an endoscope (not shown) with a fiber optic head light for viewing the surgical site, particularly in sinus procedures or procedures in the ear or the back of the mouth. The endoscope may be integral with probe 10, or it may be part of a separate instrument. The system 11 may also include a vacuum source (not shown) for coupling to a suction lumen or tube 205 (see Fig. 2) in the probe 10 for aspirating the target site.

As shown, probe 10 generally includes a proximal handle 19 and an elongate shaft 18 having an array 12 of electrode terminals 58 at its distal end. A connecting cable 34 has a connector 26 for electrically coupling the electrode terminals 58 to power supply 28. The electrode terminals 58 are electrically isolated from each other and each of the terminals 58 is connected to an active or passive control network within power supply 28 by means of a plurality of individually insulated conductors (not shown). A fluid supply tube 15 is connected to a fluid tube 14 of probe 10 for supplying electrically conducting fluid 50 to the target site. Fluid supply tube 15 may be connected to a suitable pump (not shown), if desired.

Power supply 28 has an operator controllable voltage level adjustment 30 to change the applied voltage level, which is observable at a voltage level display 32. Power supply 28 also includes first, second and third foot pedals 37, 38, 39 and a cable 36 which is removably coupled to power supply 28. The foot pedals 37, 38, 39 allow the surgeon to remotely adjust the energy level applied to electrode terminals 58. In an exemplary embodiment, first foot pedal 37 is used to place the power supply into the "ablation" mode and second foot pedal 38 places power supply 28 into the "sub-ablation" mode (e.g., coagulation or contraction of tissue). The third foot pedal 39 allows the user to adjust the voltage level within the "ablation" mode. In the ablation mode, a sufficient voltage is applied to the electrode terminals to establish the requisite conditions for molecular dissociation of the tissue (i.e., vaporizing a portion of the electrically conductive fluid, ionizing charged particles within the vapor layer and accelerating these charged particles against the tissue). As discussed above, the requisite voltage level for ablation will vary depending on the number, size, shape and spacing of the electrodes, the distance in which the electrodes extend from the support member, etc. Once the surgeon places the power supply in the "ablation" mode, voltage level adjustment 30 or third foot pedal 39 may be used to adjust the voltage level to adjust the degree or aggressiveness of the ablation.

Of course, it will be recognized that the voltage and modality of the power supply may be controlled by other input devices. However, applicant has found that foot pedals are convenient methods of controlling the power supply while manipulating the probe during a surgical procedure.

In the subablation mode, the power supply 28 applies a low enough voltage to the electrode terminals to avoid vaporization of the electrically conductive fluid and subsequent molecular dissociation of the tissue. The surgeon may automatically toggle the power supply between the ablation and sub-ablation modes by alternatively stepping on foot pedals 37, 38, respectively. In some embodiments, this allows the surgeon to quickly move between coagulation and ablation *in situ,* without having to remove his/her concentration from the surgical field or without having to request an assistant to switch the power supply. By way of example, as the surgeon is sculpting soft tissue in the ablation mode, the probe typically will simultaneously seal and/or coagulation small severed vessels within the tissue. However, larger vessels, or vessels with high fluid pressures (e.g., arterial vessels) may not be sealed in the ablation mode. Accordingly, the surgeon can simply step on foot pedal 38, automatically lowering the voltage level below the threshold level for ablation, and apply sufficient pressure onto the severed vessel for a sufficient period of time to seal and/or coagulate the vessel. After this is completed, the surgeon may quickly move back into the ablation mode by stepping on foot pedal 37. A specific design of a suitable power supply for use with the present invention can be found in U.S. Provisional Patent Application 60/062,997, filed October 23, 1997 (attorney docket no. 16238-007400).

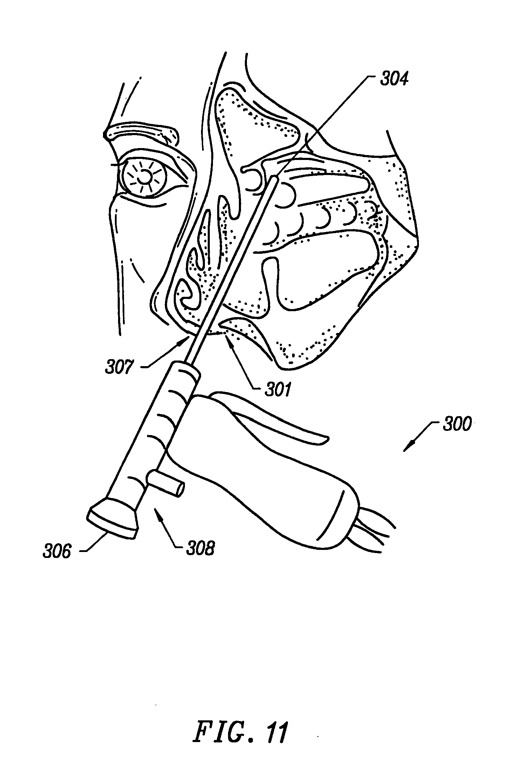

Figs. 2-5 illustrate an exemplary electrosurgical probe 90 constructed according to the principles of the present invention. As shown in Fig. 2, probe 90 generally includes an elongated shaft 100 which may be flexible or rigid, a handle 204 coupled to the proximal end of shaft 100 and an electrode support member 102 coupled to the distal end of shaft 100. Shaft 100 preferably includes a bend 101 that allows the distal section of shaft 100 to be offset from the proximal section and handle 204. This offset facilitates procedures that require an endoscope, such as FESS, because the endoscope can, for example, be introduced through the same nasal passage as the shaft 100 without interference between handle 204 and the eyepiece of the endoscope (see Fig. 11). Shaft 100 preferably comprises a plastic material that is easily molded into the shape shown in Fig. 1.

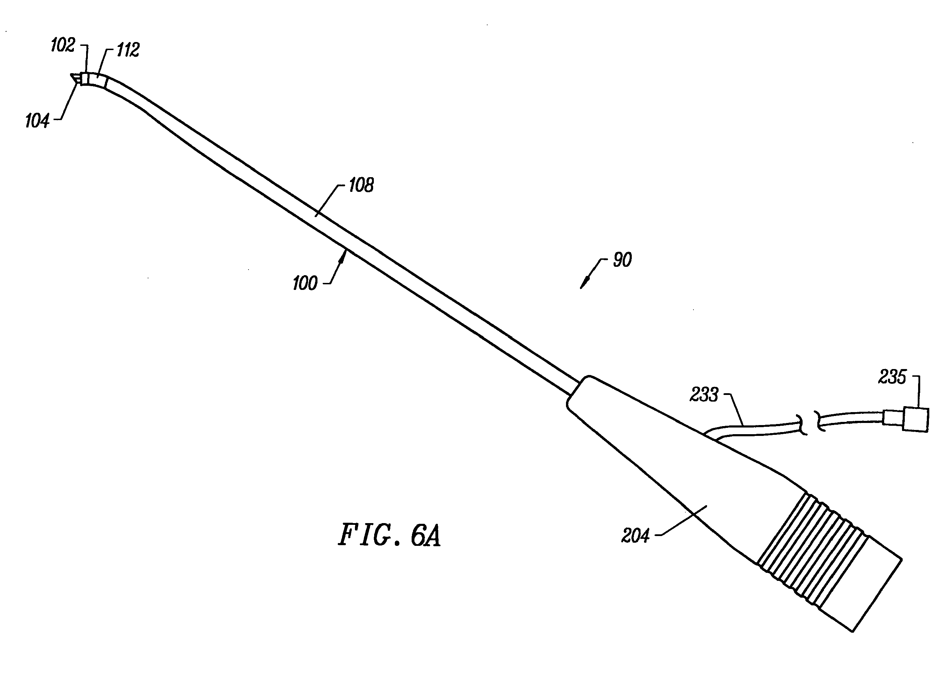

In an alternative embodiment (see Fig. 6A), shaft 100 comprises an electrically conducting material, usually metal, which is selected from the group comprising tungsten, stainless steel alloys, platinum or its alloys, titanium or its alloys, molybdenum or its alloys, and nickel or its alloys. In this embodiment, shaft 100 includes an electrically insulating jacket 108, which is typically formed as one or more electrically insulating sheaths or coatings, such as polytetrafluoroethylene, polyimide, and the like. The provision of the electrically insulating jacket over the shaft prevents direct electrical contact between these metal elements and any adjacent body structure or the surgeon. Such direct electrical contact between a body structure (e.g., tendon) and an exposed electrode could result in unwanted heating and necrosis of the structure at the point of contact causing necrosis.

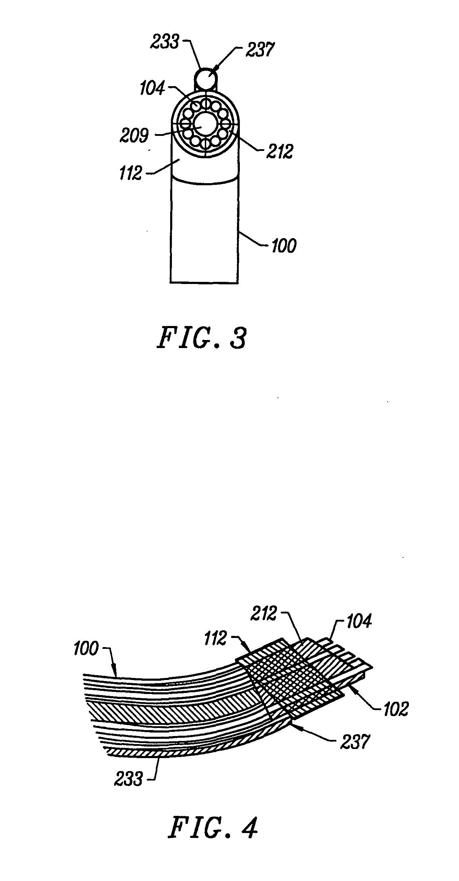

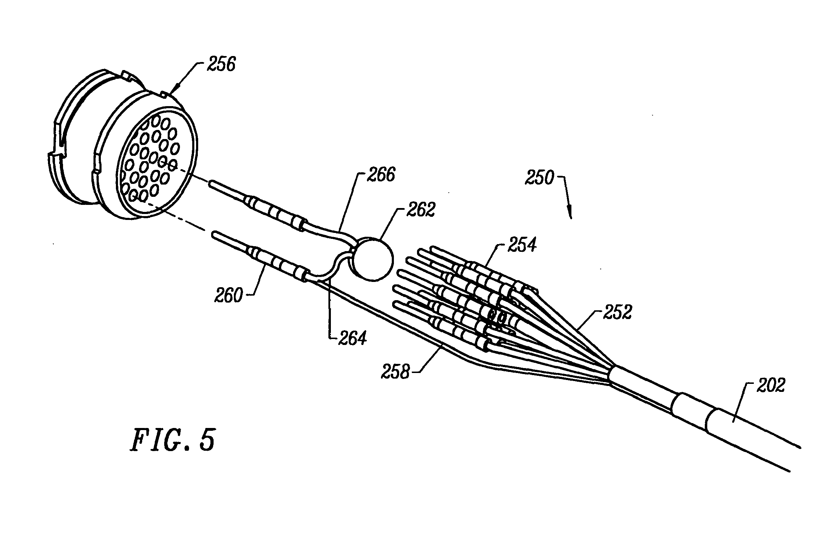

Handle 204 typically comprises a plastic material that is easily molded into a suitable shape for handling by the surgeon. Handle 204 defines an inner cavity (not shown) that houses the electrical connections 250 (Fig. 5), and provides a suitable interface for connection to an electrical connecting cable 22 (see Fig. 1). Electrode support member 102 extends from the distal end of shaft 100 (usually about 1 to 20 mm), and provides support for a plurality of electrically isolated electrode terminals 104 (see Figs. 3 and 4). As shown in Fig. 2, a fluid tube 233 extends through an opening in handle 204, and includes a connector 235 for connection to a fluid supply source, for supplying electrically conductive fluid to the target site. Depending on the configuration of the distal surface of shaft 100, fluid tube 233 may extend through a single lumen (not shown) in shaft 100, or it may be coupled to a plurality of lumens (also not shown) that extend through shaft 100 to a plurality of openings at its distal end. In the representative embodiment, fluid tube 233 extends along the exterior of shaft 100 to a point just proximal of return electrode 112 (see Fig. 4). In this embodiment, the fluid is directed through an opening 237 past return electrode 112 to the electrode terminals 104. Probe 90 may also include a valve 17 (Fig. 1) or equivalent structure for controlling the flow rate of the electrically conducting fluid to the target site.

As shown in Fig. 2, the distal portion of shaft 100 is preferably bent to improve access to the operative site of the tissue being treated. Electrode support member 102 has a substantially planar tissue treatment surface 212 (Figs. 5A and 5B) that is usually at an angle of about 10 to 90 degrees relative to the longitudinal axis of shaft 100, preferably about 30 to 60 degrees and more preferably about 45 degrees. In alternative embodiments, the distal portion of shaft 100 comprises a flexible material which can be deflected relative to the longitudinal axis of the shaft. Such deflection may be selectively induced by mechanical tension of a pull wire, for example, or by a shape memory wire that expands or contracts by externally applied temperature changes. A more complete description of this embodiment can be found in PCT International Application, U.S. National Phase Serial No. PCT/US94/05168, filed on May 10, 1994 (Attorney Docket 16238-000440).

The bend in the distal portion of shaft 100 is particularly advantageous in the treatment of sinus tissue as it allows the surgeon to reach the target tissue within the nose as the shaft 100 extends through the nasal passage. Of course, it will be recognized that the shaft may have different angles depending on the procedure. For example, a shaft having a 90° bend angle may be particularly useful for accessing tissue located in the back portion of the mouth and a shaft having a 10° to 30° bend angle may be useful for accessing tissue near or in the front portion of the mouth or nose

In the embodiment shown in Figs. 2-5, probe 90 includes a return electrode 112 for completing the current path between electrode terminals 104 and a high frequency power supply 28 (see Fig. 1). As shown, return electrode 112 preferably comprises an annular conductive band coupled to the distal end of shaft 100 slightly proximal to tissue treatment surface 212 of electrode support member 102, typically about 0.5 to 10 mm and more preferably about 1 to 10 mm. Return electrode 112 is coupled to a connector 258 that extends to the proximal end of probe 10, where it is suitably connected to power supply 10 (Fig. 1).

As shown in Fig. 2, return electrode 112 is not directly connected to electrode terminals 104. To complete this current path so that electrode terminals 104 are electrically connected to return electrode 112, electrically conducting fluid (e.g., isotonic saline) is caused to flow therebetween. In the representative embodiment, the electrically conducting fluid is delivered through fluid tube 233 to opening 237, as described above. Alternatively, the fluid may be delivered by a fluid delivery element (not shown) that is separate from probe 90. In arthroscopic surgery, for example, the body cavity will be flooded with isotonic saline and the probe 90 will be introduced into this flooded cavity. Electrically conducting fluid will be continually resupplied to maintain the conduction path between return electrode 112 and electrode terminals 104.

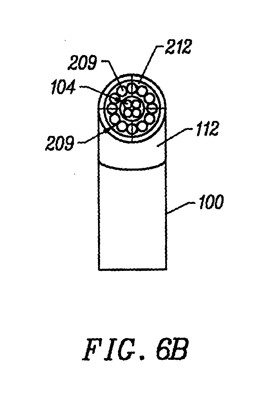

In alternative embodiments, the fluid path may be formed in probe 90 by, for example, an inner lumen or an annular gap between the return electrode and a tubular support member within shaft 100 (see Fig. 6). This annular gap may be formed near the perimeter of the shaft 100 such that the electrically conducting fluid tends to flow radially inward towards the target site, or it may be formed towards the center of shaft 100 so that the fluid flows radially outward. In both of these embodiments, a fluid source (e.g., a bag of fluid elevated above the surgical site or having a pumping device), is coupled to probe 90 via a fluid supply tube (not shown) that may or may not have a controllable valve. A more complete description of an electrosurgical probe incorporating one or more fluid lumen(s) can be found in parent application Serial No. 08/485,219, filed on June 7, 1995 (Attorney Docket 16238-006000).

Referring to Fig. 3, the electrically isolated electrode terminals 104 are spaced apart over tissue treatment surface 212 of electrode support member 102. The tissue treatment surface and individual electrode terminals 104 will usually have dimensions within the ranges set forth above. In the representative embodiment, the tissue treatment surface 212 has an oval cross-sectional shape with a length in the range of 1 mm to 20 mm and a width in the range from 0.3 mm to 7 mm. The oval cross-sectional shape accommodates the bend in the distal portion of shaft 100. The individual electrode terminals 104 preferably extend outward from tissue treatment surface 212 by a distance of about 0.1 to 4 mm, usually about 0.2 to 2 mm. Applicant has found that this configuration increases the high electric field intensities and associated current densities around electrode terminals 104 to facilitate the ablation of tissue as described in detail above.

In the embodiment of Figs. 2-5, the probe includes a single, larger opening 209 in the center of tissue treatment surface 212, and a plurality of electrode terminals (e.g., about 3-15) around the perimeter of surface 212 (see Fig. 3). Alternatively, the probe may include a single, annular, or partially annular, electrode terminal at the perimeter of the tissue treatment surface. The central opening 209 is coupled to a suction lumen (not shown) within shaft 100 and a suction tube 211 (Fig. 2) for aspirating tissue, fluids and/or gases from the target site. In this embodiment, the electrically conductive fluid generally flows radially inward past electrode terminals 104 and then back through the opening 209. Aspirating the electrically conductive fluid during surgery allows the surgeon to see the target site, and it prevents the fluid from flowing into the patient's body, e.g., through the sinus passages, down the patient's throat or into the ear canal.

Of course, it will be recognized that the distal tip of probe may have a variety of different configurations. For example, the probe may include a plurality of openings 209 around the outer perimeter of tissue treatment surface 212 (see Fig. 6B). In this embodiment, the electrode terminals 104 extend from the center of tissue treatment surface 212 radially inward from openings 209. The openings are suitably coupled to fluid tube 233 for delivering electrically conductive fluid to the target site, and suction tube 211 for aspirating the fluid after it has completed the conductive path between the return electrode 112 and the electrode terminals 104.

Fig. 5 illustrates the electrical connections 250 within handle 204 for coupling electrode terminals 104 and return electrode 112 to the power supply 28. As shown, a plurality of wires 252 extend through shaft 100 to couple terminals 104 to a plurality of pins 254, which are plugged into a connector block 256 for coupling to a connecting cable 22 (Fig. 1). Similarly, return electrode 112 is coupled to connector block 256 via a wire 258 and a plug 260.

According to the present invention, the probe 90 further includes an identification element that is characteristic of the particular electrode assembly so that the same power supply 28 can be used for different electrosurgical operations. In one embodiment, for example, the probe 90 includes a voltage reduction element or a voltage reduction circuit for reducing the voltage applied between the electrode terminals 104 and the return electrode 112. The voltage reduction element serves to reduce the voltage applied by the power supply so that the voltage between the electrode terminals and the return electrode is low enough to avoid excessive power dissipation into the electrically conducting medium and/or ablation of the soft tissue at the target site. The voltage reduction element primarily allows the electrosurgical probe 90 to be compatible with other ArthroCare generators that are adapted to apply higher voltages for ablation or vaporization of tissue. For contraction of tissue, for example, the voltage reduction element will serve to reduce a voltage of about 100 to 135 volts rms (which is a setting of 1 on the ArthroCare Model 970 and 980 (i.e., 2000) Generators) to about 45 to 60 volts rms, which is a suitable voltage for contraction of tissue without ablation (e.g., molecular dissociation) of the tissue.

Of course, for some procedures, such as endoscopic sinus surgery, the probe will typically not require a voltage reduction element. Alternatively, the probe may include a voltage increasing element or circuit, if desired.

In the representative embodiment, the voltage reduction element is a dropping capacitor 262 which has first leg 264 coupled to the return electrode wire 258 and a second leg 266 coupled to connector block 256. Of course, the capacitor may be located in other places within the system, such as in, or distributed along the length of, the cable, the generator, the connector, etc. In addition, it will be recognized that other voltage reduction elements, such as diodes, transistors, inductors, resistors, capacitors or combinations thereof, may be used in conjunction with the present invention. For example, the probe 90 may include a coded resistor (not shown) that is constructed to lower the voltage applied between return electrode 112 and electrode terminals 104 to a suitable level for contraction of tissue. In addition, electrical circuits may be employed for this purpose.

Alternatively or additionally, the cable 22 that couples the power supply 10 to the probe 90 may be used as a voltage reduction element. The cable has an inherent capacitance that can be used to reduce the power supply voltage if the cable is placed into the electrical circuit between the power supply, the electrode terminals and the return electrode. In this embodiment, the cable 22 may be used alone, or in combination with one of the voltage reduction elements discussed above, e.g., a capacitor.

Further, it should be noted that the present invention can be used with a power supply that is adapted to apply a voltage within the selected range for treatment of tissue. In this embodiment, a voltage reduction element or circuitry may not be desired.

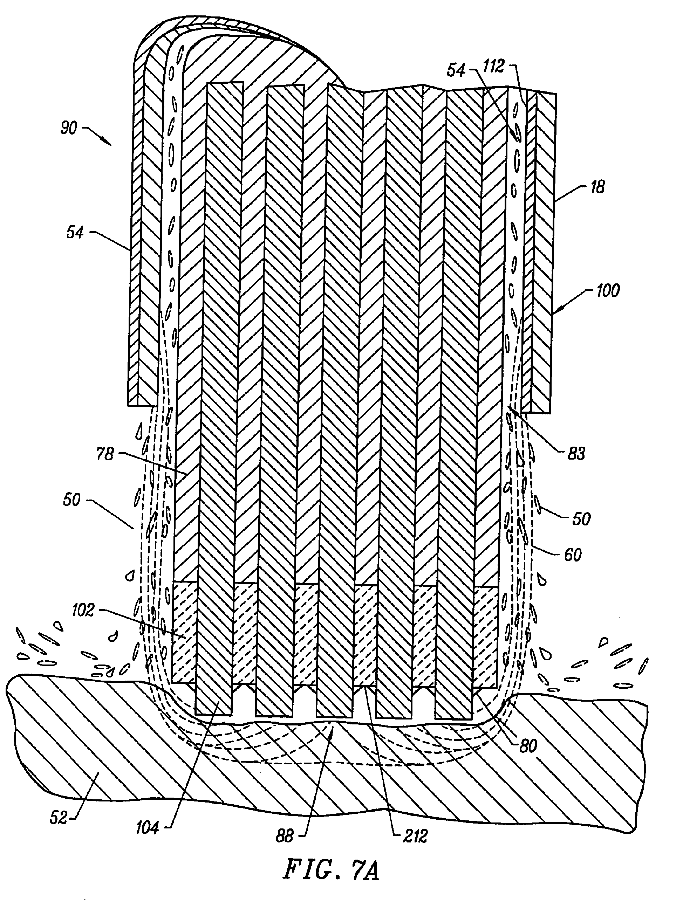

Figs. 7A-7C schematically illustrate the distal portion of three different embodiments of probe 90 according to the present invention. As shown in 7A, electrode terminals 104 are anchored in a support matrix 102 of suitable insulating material (e.g., ceramic or glass material, such as alumina, zirconia and the like) which could be formed at the time of manufacture in a flat, hemispherical or other shape according to the requirements of a particular procedure. The preferred support matrix material is alumina, available from Kyocera Industrial Ceramics Corporation, Elkgrove, Illinois, because of its high thermal conductivity, good electrically insulative properties, high flexural modulus, resistance to carbon tracking, biocompatibility, and high melting point. The support matrix 102 is adhesively joined to a tubular support member 78 that extends most or all of the distance between matrix 102 and the proximal end of probe 90. Tubular member 78 preferably comprises an electrically insulating material, such as an epoxy or silicone-based material.

In a preferred construction technique, electrode terminals 104 extend through pre-formed openings in the support matrix 102 so that they protrude above tissue treatment surface 212 by the desired distance. The electrodes are then bonded to the tissue treatment surface 212 of support matrix 102, typically by an inorganic sealing material 80. Sealing material 80 is selected to provide effective electrical insulation, and good adhesion to both the alumina matrix 102 and the platinum or titanium electrode terminals. Sealing material 80 additionally should have a compatible thermal expansion coefficient and a melting point well below that of platinum or titanium and alumina or zirconia, typically being a glass or glass ceramic.

In the embodiment shown in Fig. 7A, return electrode 112 comprises an annular member positioned around the exterior of shaft 100 of probe 90. Return electrode 90 may fully or partially circumscribe tubular support member 78 to form an annular gap 54 therebetween for flow of electrically conducting liquid 50 therethrough, as discussed below. Gap 54 preferably has a width in the range of 0.25 mm to 4 mm. Alternatively, probe may include a plurality of longitudinal ribs between support member 78 and return electrode 112 to form a plurality of fluid lumens extending along the perimeter of shaft 100. In this embodiment, the plurality of lumens will extend to a plurality of openings.

Return electrode 112 is disposed within an electrically insulative jacket 18, which is typically formed as one or more electrically insulative sheaths or coatings, such as polytetrafluoroethylene, polyamide, and the like. The provision of the electrically insulative jacket 18 over return electrode 112 prevents direct electrical contact between return electrode 56 and any adjacent body structure. Such direct electrical contact between a body structure (e.g., tendon) and an exposed electrode member 112 could result in unwanted heating and necrosis of the structure at the point of contact causing necrosis.

As shown in Fig. 7A, return electrode 112 is not directly connected to electrode terminals 104. To complete this current path so that terminals 104 are electrically connected to return electrode 112, electrically conducting liquid 50 (e.g., isotonic saline) is caused to flow along fluid path(s) 83. Fluid path 83 is formed by annular gap 54 between outer return electrode 112 and tubular support member. The electrically conducting liquid 50 flowing through fluid path 83 provides a pathway for electrical current flow between electrode terminals 104 and return electrode 112, as illustrated by the current flux lines 60 in Fig. 6A. When a voltage difference is applied between electrode terminals 104 and return electrode 112, high electric field intensities will be generated at the distal tips of terminals 104 with current flow from terminals 104 through the target tissue to the return electrode, the high electric field intensities causing ablation of tissue 52 in zone 88.

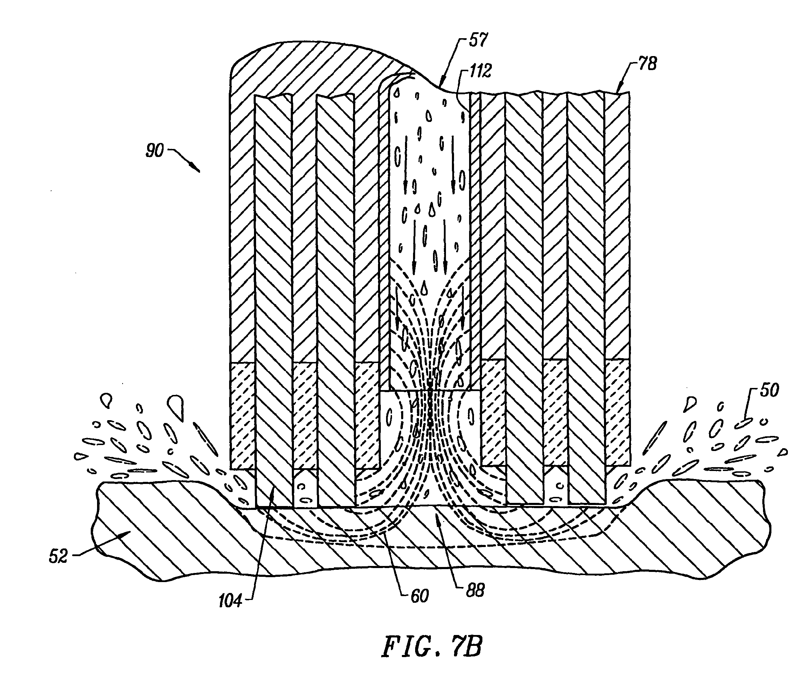

Fig. 7B illustrates another alternative embodiment of electrosurgical probe 90 which has a return electrode 112 positioned within tubular member 78. Return electrode 112 is preferably a tubular member defining an inner lumen 57 for allowing electrically conducting liquid 50 (e.g., isotonic saline) to flow therethrough in electrical contact with return electrode 112. In this embodiment, a voltage difference is applied between electrode terminals 104 and return electrode 112 resulting in electrical current flow through the electrically conducting liquid 50 as shown by current flux lines 60 (Fig. 3). As a result of the applied voltage difference and concomitant high electric field intensities at the tips of electrode terminals 104, tissue 52 becomes ablated or transected in zone 88.

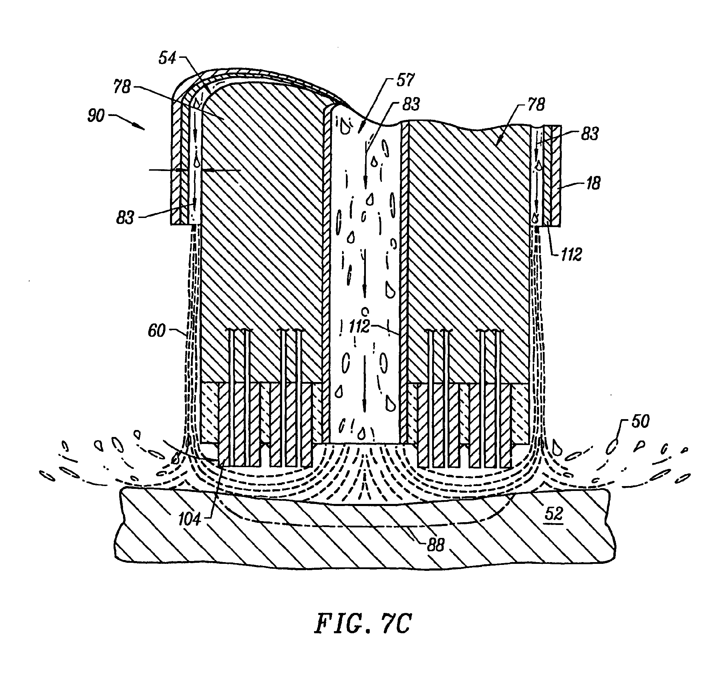

Fig. 7C illustrates another embodiment of probe 90 that is a combination of the embodiments in Figs. 7A and 7B. As shown, this probe includes both an inner lumen 57 and an outer gap or plurality of outer lumens 54 for flow of electrically conductive fluid. In this embodiment, the return electrode 112 may be positioned within tubular member 78 as in Fig. 7B, outside of tubular member 78 as in Fig. 7A, or in both locations.

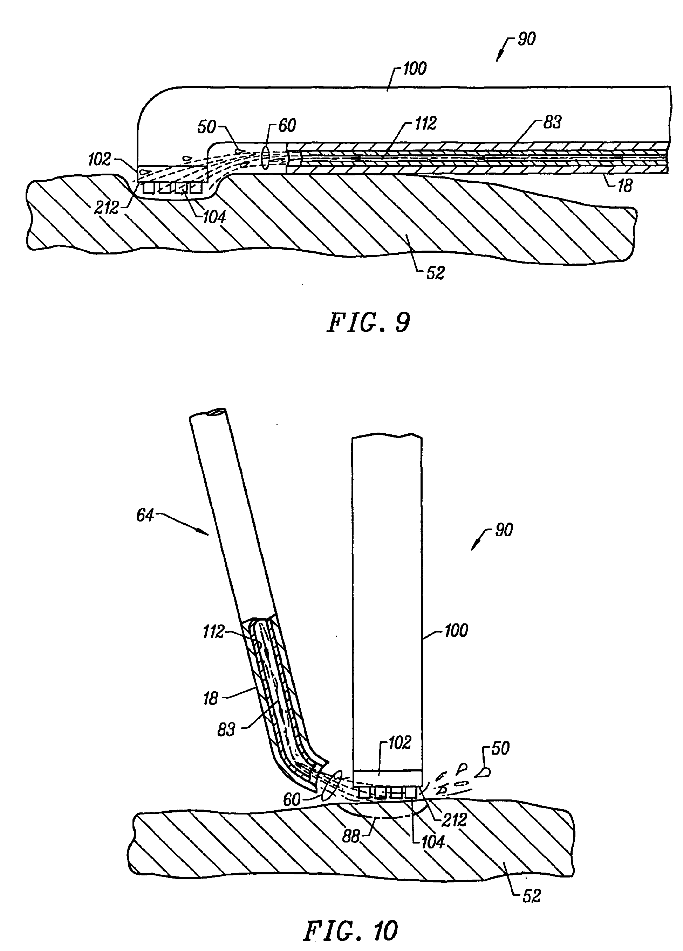

Fig. 9 illustrates another embodiment of probe 90 where the distal portion of shaft 100 is bent so that electrode terminals extend transversely to the shaft. Preferably, the distal portion of shaft 100 is perpendicular to the rest of the shaft so that tissue treatment surface 212 is generally parallel to the shaft axis. In this embodiment, return electrode 112 is mounted to the outer surface of shaft 100 and is covered with an electrically insulating jacket 18. The electrically conducting fluid 50 flows along flow path 83 through return electrode 112 and exits the distal end of electrode 112 at a point proximal of tissue treatment surface 212. The fluid is directed exterior of shaft to surface 212 to create a return current path from electrode terminals 104, through the fluid 50, to return electrode 12, as shown by current flux lines 60.

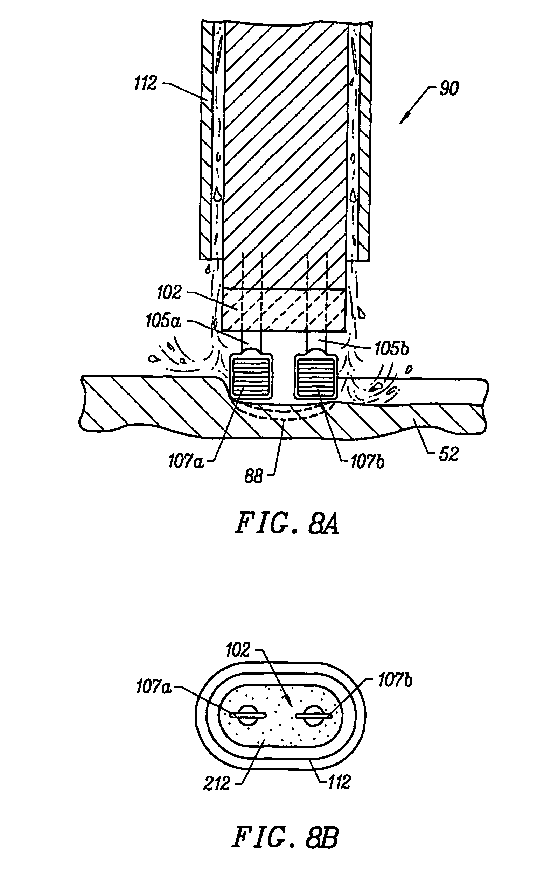

Fig. 10 illustrates another embodiment of the invention where electrosurgical system 11 further includes a liquid supply instrument 64 for supplying electrically conducting fluid 50 between electrode terminals 104 and return electrode 112. Liquid supply instrument 64 comprises an inner tubular member or return electrode 112 surrounded by an electrically insulating jacket 18. Return electrode 112 defines an inner passage 83 for flow of fluid 50. As shown in Fig. 8, the distal portion of instrument 64 is preferably bent so that liquid 50 is discharged at an angle with respect to instrument 64. This allows the surgical team to position liquid supply instrument 64 adjacent tissue treatment surface 212 with the proximal portion of supply instrument 64 oriented at a similar angle to probe 90.

The present invention is not limited to an electrode array disposed on a relatively planar surface at the distal tip of probe 90, as described above. Referring to Figs. 8A and 8B, an alternative probe 90 includes a pair of electrodes 105a, 105b mounted to the distal end of shaft 100. Electrodes 105a, 105b are electrically connected to power supply as described above and preferably have tips 107a, 107b with a screwdriver shape. The screwdriver shape provides a greater amount of "edges" to electrodes 105a, 105b, to increase the electric field intensity and current density at the edges and thereby improve the cutting ability as well as the ability to limit bleeding from the incised tissue (i.e., hemostasis).

Figs. 11-13 illustrate a method for treating nasal or sinus blockages, e.g., chronic sinusitis, according to the present invention. In these procedures, the polyps, turbinates or other sinus tissue may be ablated or reduced (e.g., by tissue contraction) to clear the blockage and/or enlarge the sinus cavity to reestablish normal sinus function. For example, in chronic rhinitis, which is a collective term for chronic irritation or inflammation of the nasal mucosa with hypertrophy of the nasal mucosa, the inferior turbinate may be reduced by ablation or contraction. Alternatively, a turbinectomy or mucotomy may be performed by removing a strip of tissue from the lower edge of the inferior turbinate to reduce the volume of the turbinate. For treating nasal polypi, which comprises benign pedicled or sessile masses of nasal or sinus mucosa caused by inflammation, the nasal polypi may be contracted or shrunk, or ablated by the method of the present invention. For treating severe sinusitis, a frontal sinus operation may be performed to introduce the electrosurgical probe to the site of blockage. The present invention may also be used to treat diseases of the septum, e.g., ablating or resecting portions of the septum for removal, straightening or reimplantation of the septum.

The present invention is particularly useful in functional endoscopic sinus surgery (FESS) in the treatment of sinus disease. In contrast to prior art microdebriders, the electrosurgical probe of the present invention effects hemostasis of severed blood vessels, and allows the surgeon to precisely remove tissue with minimal or no damage to surrounding tissue, bone, cartilage or nerves. By way of example and not limitation, the present invention may be used for the following procedures: (1) uncinectomy or medial displacement or removal of portions of the middle turbinate; (2) maxillary, sphenoid or ethmoid sinusotomies or enlargement of the natural ostium of the maxillary, sphenoid, or ethmoid sinuses, respectively; (3) frontal recess dissections, in which polypoid or granulation tissue are removed; (4) polypectomies, wherein polypoid tissue is removed in the case of severe nasal polyposis; (5) concha bullosa resections or the thinning of polypoid middle turbinate; (6) septoplasty; and the like.

Figures 11-13 schematically illustrate an endoscopic sinus surgery (FESS) procedure according to the present invention. As shown in Fig. 11, an endoscope 300 is first introduced through one of the nasal passages 301 to allow the surgeon to view the target site, e.g., the sinus cavities. As shown, the endoscope 300 will usually comprise a thin metal tube 302 with a lens (not shown) at the distal end 304, and an eyepiece 306 at the proximal end 308. As shown in Fig. 2, the probe shaft 100 (not shown in Fig. 11) has a bend 101 to facilitate use of both the endoscope and the probe 90 in the same nasal passage (i.e., the handles of the two instruments do not interfere with each other in this embodiment). Alternatively, the endoscope may be introduced transorally through the inferior soft palate to view the nasopharynx. Suitable nasal endoscopes for use with the present invention are described in U.S. Patent Nos. 4,517,962, 4,844,052, 4,881,523 and 5,167,220, the complete disclosures of which are incorporated herein by reference for all purposes.

Alternatively, the endoscope 300 may include a sheath (not shown) having an inner lumen for receiving the electrosurgical probe shaft 100. In this embodiment, the shaft 100 will extend through the inner lumen to a distal opening in the endoscope. The shaft will include suitable proximal controls for manipulation of its distal end during the surgical procedure.

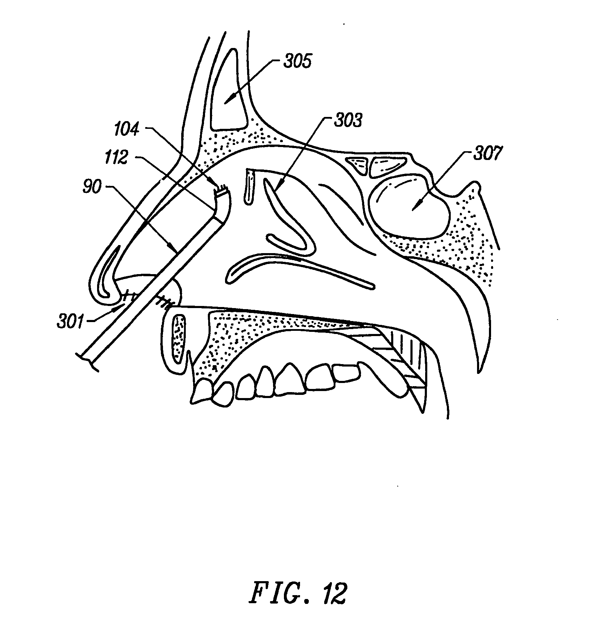

As shown in Fig. 12, the distal end of probe 90 is introduced through nasal passage 301 into the nasal cavity 303 (endoscope 300 is not shown in Fig. 12). Depending on the location of the blockage, the electrode terminals 104 will be positioned adjacent the blockage in the nasal cavity 303, or in one of the paranasal sinuses 305, 307. Note that only the frontal sinus 305 and the sphenoidal sinus 307 are shown in Fig. 12, but the procedure is also applicable to the ethmoidal and maxillary sinuses. Once the surgeon has reached the point of major blockage, electrically conductive fluid is delivered through tube 233 and opening 237 to the tissue (see Fig. 2). The fluid flows past the return electrode 112 to the electrode terminals 104 at the distal end of the shaft. The rate of fluid flow is controlled with valve 17 (Fig. 1) such that the zone between the tissue and electrode support 102 is constantly immersed in the fluid. The power supply 28 is then turned on and adjusted such that a high frequency voltage difference is applied between electrode terminals 104 and return electrode 112. The electrically conductive fluid provides the conduction path (see current flux lines) between electrode terminals 104and the return electrode 112.

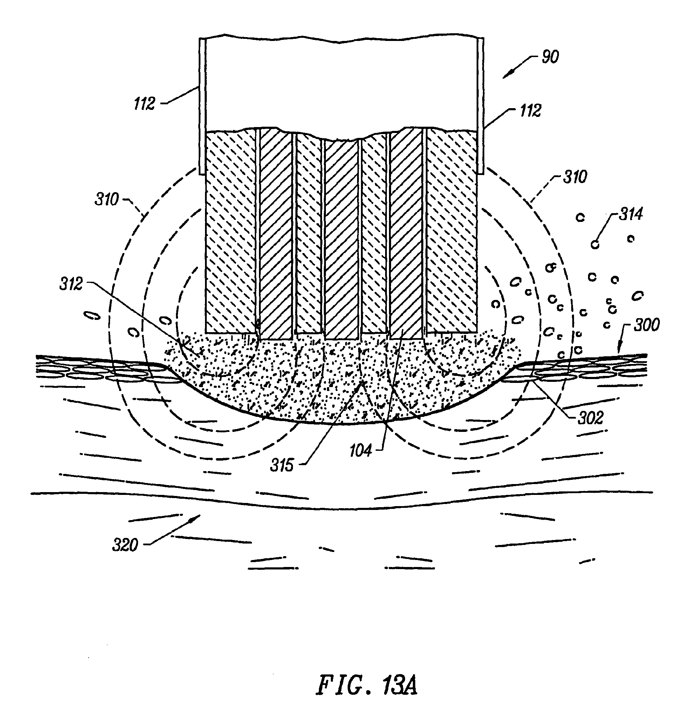

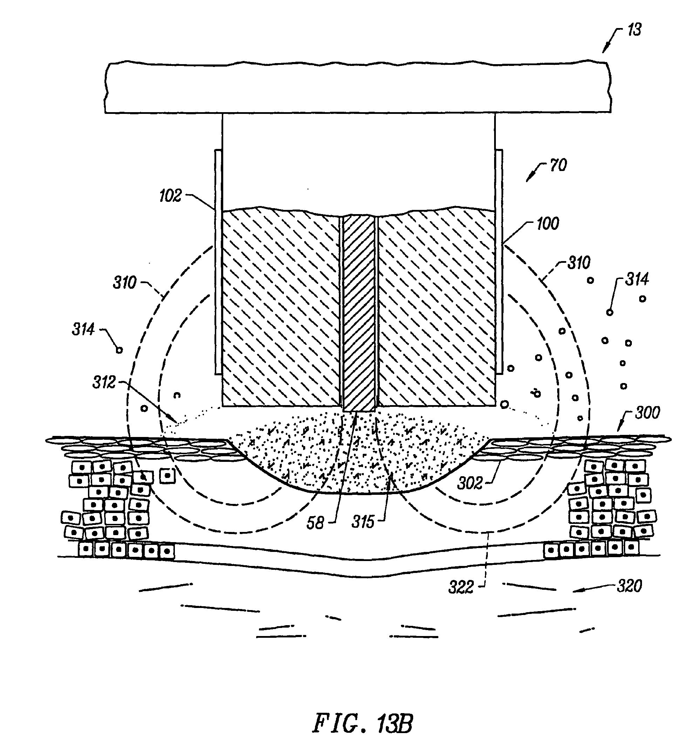

Figs. 13A and 13B illustrate the removal of sinus tissue in more detail As shown, the high frequency voltage is sufficient to convert the electrically conductive fluid (not shown) between the target tissue 302 and electrode terminal(s)104 into an ionized vapor layer 312 or plasma. As a result of the applied voltage difference between electrode terminal(s) 104 and the target tissue 302 (i.e., the voltage gradient across the plasma layer 312), charged particles 315 in the plasma (viz., electrons) are accelerated towards the tissue. At sufficiently high voltage differences, these charged particles 315 gain sufficient energy to cause dissociation of the molecular bonds within tissue structures. This molecular dissociation is accompanied by the volumetric removal (i.e., ablative sublimation) of tissue and the production of low molecular weight gases 314, such as oxygen, nitrogen, carbon dioxide, hydrogen and methane. The short range of the accelerated charged particles 315 within the tissue confines the molecular dissociation process to the surface layer to minimize damage and necrosis to the underlying tissue 320.

During the process, the gases 314 will be aspirated through opening 209 and suction tube 211 to a vacuum source. In addition, excess electrically conductive fluid, and other fluids (e.g., blood) will be aspirated from the target site 300 to facilitate the surgeon's view. During ablation of the tissue, the residual heat generated by the current flux lines (typically less than 150°C), will usually be sufficient to coagulate any severed blood vessels at the site. If not, the surgeon may switch the power supply 28 into the coagulation mode by lowering the voltage to a level below the threshold for fluid vaporization, as discussed above. This simultaneous hemostasis results in less bleeding and facilitates the surgeon's ability to perform the procedure. Once the blockage has been removed, aeration and drainage are reestablished to allow the sinuses to heal and return to their normal function.

Another advantage of the present invention is the ability to precisely ablate layers of sinus tissue without causing necrosis or thermal damage to the underlying and surrounding tissues, nerves (e.g., the optic nerve) or bone. In addition, the voltage can be controlled so that the energy directed to the target site is insufficient to ablate bone or adipose tissue (which generally has a higher impedance than the target sinus tissue). In this manner, the surgeon can literally clean the tissue off the bone, without ablating or otherwise effecting significant damage to the bone.

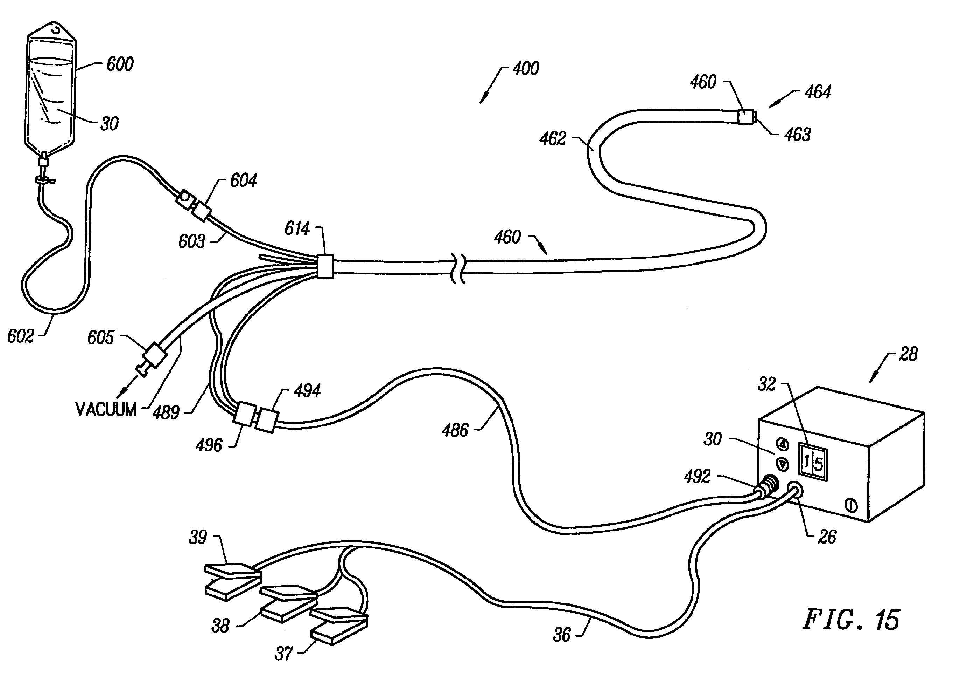

Referring to Figs. 15-17, the electrosurgical device according to the present invention may also be configured as a catheter system 400. As shown in Fig. 15, a catheter system 400 generally comprises an electrosurgical catheter 460 connected to a power supply 28 by an interconnecting cable 486 for providing high frequency voltage to a target tissue and an irrigant reservoir or source 600 for providing electrically conducting fluid to the target site. Catheter 460 generally comprises an elongate, flexible shaft body 462 including a tissue removing or ablating region 464 at the distal end of body 462. The proximal portion of catheter 460 includes a multi-lumen fitment 614 which provides for interconnections between lumens and electrical leads within catheter 460 and conduits and cables proximal to fitment 614. By way of example, a catheter electrical connector 496 is removably connected to a distal cable connector 494 which, in turn, is removably connectable to generator 28 through connector 492. One or more electrically conducting lead wires (not shown) within catheter 460 extend between one or more active electrodes 463 at tissue ablating region 464 and one or more corresponding electrical terminals (also not shown) in catheter connector 496 via active electrode cable branch 487. Similarly, one or more return electrodes 466 at tissue ablating region 464 are coupled to a return electrode cable branch 489 of catheter connector 496 by lead wires (not shown). Of course, a single cable branch (not shown) may be used for both active and return electrodes.

Catheter body 462 may include reinforcing fibers or braids (not shown) in the walls of at least the distal ablation region 464 of body 462 to provide responsive torque control for rotation of electrode terminals during tissue engagement. This rigid portion of the catheter body 462 preferably extends only about 7 to 10 mm while the remainder of the catheter body 462 is flexible to provide good trackability during advancement and positioning of the electrodes adjacent target tissue.

Conductive fluid 30 is provided to tissue ablation region 464 of catheter 460 via a lumen (not shown in Fig. 15) within catheter 460. Fluid is supplied to lumen from the source along a conductive fluid supply line 602 and a conduit 603, which is coupled to the inner catheter lumen at multi-lumen fitment 114. The source of conductive fluid (e.g., isotonic saline) may be an irrigant pump system (not shown) or a gravity-driven supply, such as an irrigant reservoir 600 positioned several feet above the level of the patient and tissue ablating region 8. A control valve 604 may be positioned at the interface of fluid supply line 602 and conduit 603 to allow manual control of the flow rate of electrically conductive fluid 30. Alternatively, a metering pump or flow regulator may be used to precisely control the flow rate of the conductive fluid.

System 400 further includes an aspiration or vacuum system (not shown) to aspirate liquids and gases from the target site. The aspiration system will usually comprise a source of vacuum coupled to fitment 614 by a aspiration connector 605.

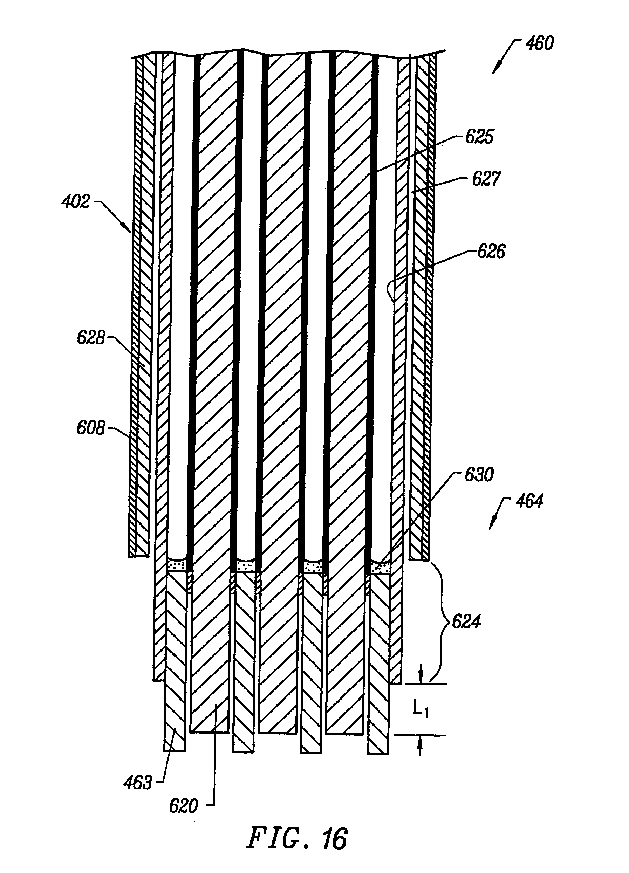

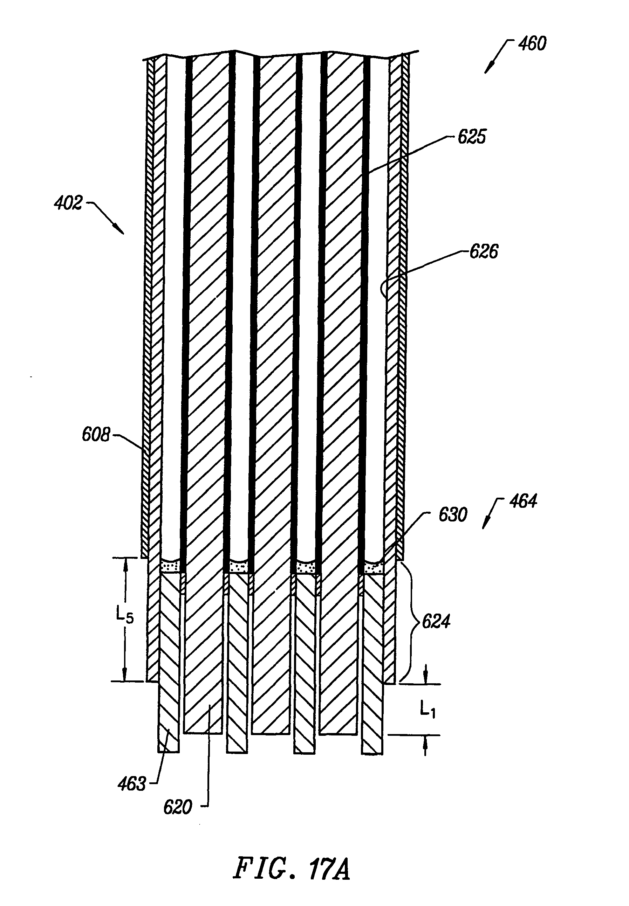

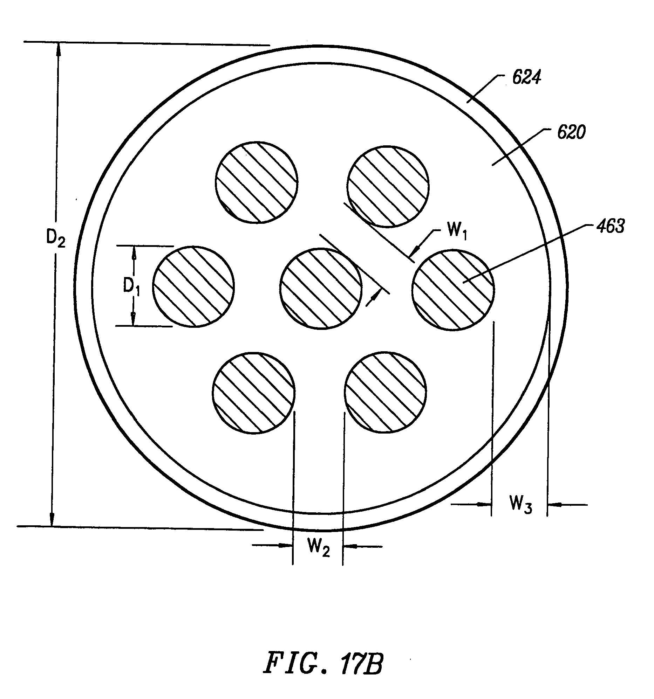

Figs. 16 and 17 illustrate the working end 464 of an electrosurgical catheter 460 constructed according to the principles of the present invention. As shown in Fig. 16, catheter 460 generally includes an elongated shaft 462 which may be flexible or rigid, and an electrode support member 620 coupled to the distal end of shaft 462. Electrode support member 620 extends from the distal end of shaft 462 (usually about 1 to 20 mm), and provides support for a plurality of electrically isolated electrode terminals 463. Electrode support member 620 and electrode terminals 462 are preferably secured to a tubular support member 626 within shaft 460 by adhesive 630.

The electrode terminals 463 may be constructed using round, square, rectangular or other shaped conductive metals. By way of example, the electrode terminal materials may be selected from the group including stainless steel, tungsten and its alloys, molybdenum and its alloys, titanium and its alloys, nickel-based alloys, as well as platinum and its alloys. Electrode support member 620 is preferably a ceramic, glass or glass/ceramic composition (e.g., aluminum oxide, titanium nitride). Alternatively, electrode support member 620 may include the use of high-temperature biocompatible plastics such as polyether-ether-keytone (PEEK) manufactured by Vitrex International Products, Inc. or polysulfone manufactured by GE Plastics. The adhesive 630 may, by way of example, be an epoxy (e.g., Master Bond EP42HT) or a silicone-based adhesive.

As shown in Fig. 17B, a total of 7 circular active electrodes or electrode terminals 463 are shown in a symmetrical pattern having an active electrode diameter, D₁ in the range from 0.05 mm to 1.5 mm, more preferably in the range from 0.1 mm to 0.75 mm. The interelectrode spacings, W₁ and W₂ are preferably in the range from 0.1 mm to 1.5 mm and more preferably in the range from 0.2 mm to 0.75 mm. The distance between the outer perimeter of the electrode terminal 463 and the perimeter of the electrode support member, W₃ is preferably in the range from 0.1 mm to 1.5 mm and more preferably in the range from 0.2 mm to 0.75 mm. The overall diameter, D₂ of the working end 464 of catheter body 462 is preferably in the range from 0.5 mm to 10 mm and more preferably in the range from 0.5 mm to 5 mm. As discussed above, the shape of the active electrodes may be round , square, triangular, hexagonal, rectangular, tubular, flat strip and the like and may be arranged in a circularly symmetric pattern or may, by way of example, be arranged in a rectangular pattern, square pattern, or strip.

Catheter body 462 includes a tubular cannula 626 extending along body 462 radially outward from support member 620 and electrode terminals 463. The material for cannula 626 may be advantageously selected from a group of electrically conductive metals so that the cannula 626 functions as both a structural support member for the array of electrode terminals 463 as well as a return electrode 624. The support member 626 is connected to an electrical lead wire (not shown) at its proximal end within a connector housing (not shown) and continues via a suitable connector to power supply 28 to provide electrical continuity between one output pole of high frequency generator 28 and said return electrode 624. The cannula 626 may be selected from the group including stainless steel, copper-based alloys, titanium or its alloys, and nickel-based alloys. The thickness of the cannula 626 is preferably in the range from 0.08 mm to 1.0 mm and more preferably in the range from 0.1 mm to 0.4 mm.

As shown in Fig. 16, cannula 626 is covered with an electrically insulating sleeve 608 to protect the patient's body from the electric current. Electrically insulating sleeve 608 may be a coating (e.g., nylon) or heat shrinkable plastic (e.g., fluropolymer or polyester). The proximal portion of the cannula 626 is left exposed to function as the return electrode 624. The length of the return electrode 624, L₅ is preferably in the range from 1 mm to 30 mm and more preferably in the range from 2 mm to 20 mm. The spacing between the most distal portion of the return electrode 624 and the plane of the tissue treatment surface 622 of the electrode support member 620, L₁ is preferably in the range from 0.5 mm to 30 mm and more preferably in the range from 1 mm to 20 mm. The thickness of the electrically insulating sleeve 608 is preferably in the range from 0.01 mm to 0.5 mm and more preferably in the range from 0.02 mm to 0.2 mm.

In the representative embodiment, the fluid path is formed in catheter by an inner lumen 627 or annular gap between the return electrode 624 and a second tubular support member 628 within shaft 460. This annular gap may be formed near the perimeter of the shaft 460 as shown in Fig. 16 such that the electrically conducting fluid tends to flow radially inward towards the target site, or it may be formed towards the center of shaft 460 (not shown) so that the fluid flows radially outward. In both of these embodiments, a fluid source (e.g., a bag of fluid elevated above the surgical site or having a pumping device), is coupled to catheter 460 via a fluid supply tube (not shown) that may or may not have a controllable valve.

In an alternative embodiment shown in Fig. 17A, the electrically conducting fluid is delivered from a fluid delivery element (not shown) that is separate from catheter 460. In arthroscopic surgery, for example, the body cavity will be flooded with isotonic saline and the catheter 460 will be introduced into this flooded cavity. Electrically conducting fluid will be continually resupplied to maintain the conduction path between return electrode 624 and electrode terminals 463.

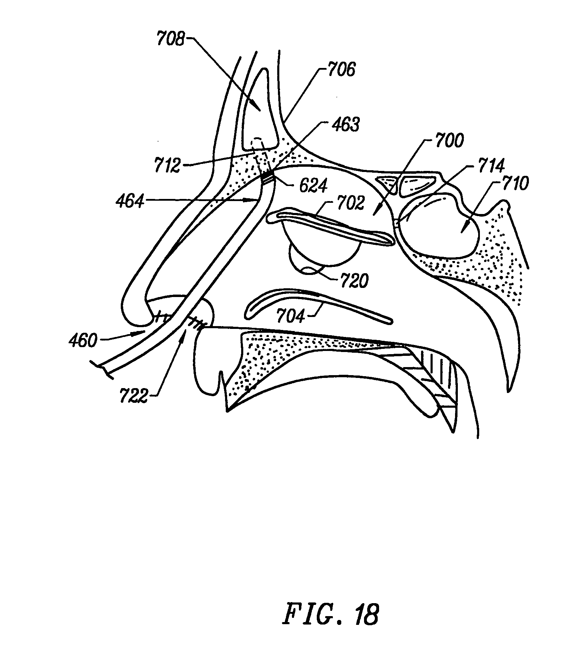

Fig. 18 illustrates a method of removing tissue from a body lumen within a patient's nose. As shown, the patient's nose includes a nasal cavity 700 divided up by the inferior and middle nasal conchas 702, 704, and a number of sinus cavities separated from the nasal cavity by ethmoid bone 706. As shown, the frontal sinus 708 and the sphenoidal sinus 710 each include a passage or lumen 712, 714, respectively, extending through ethmoid bone 706 into the nasal cavity 700. In addition, the sinus cavities have passages (not shown) that connect each other. These passages often become blocked with swollen or scarred tissue, which causes the sinus cavities to fill, producing deep pain and pressure. Postnasal or nasal drainage, nasal congestion with pressure, headaches, sinus infections and nasal polyps are most commonly associated with chronic sinusitis. Note that only the frontal sinus 305 and the sphenoidal sinus 307 are shown in Fig. 18, but the procedure is also applicable to the ethmoidal and maxillary sinuses. The ostium 720 for the maxillary sinus is shown in Fig. 18.

As shown in Fig. 18, the ablation region 464 of catheter 460 is advanced through the nostril 722 into the nasal cavity 700 and to the passage 712 leading to the frontal sinus 708. The catheter 460 may be advanced with a variety of techniques, such as a guidewire, steerable catheter and the like. Once the surgeon has reached the point of major blockage within passage 712, electrically conductive fluid is delivered through one or more internal lumen(s) (not shown) within the catheter to the tissue. Alternatively, the nasal cavity 700 is filled with electrically conductive fluid (similar to an arthroscopic procedure). In some embodiments, the catheter may be configured to operate with a naturally occurring body fluid, e.g., blood, as the conductive medium. The fluid flows past the return electrode 624 to the electrode terminals 463 at the distal end of the catheter shaft. The rate of fluid flow is controlled with a valve (not shown) such that the zone between the occlusion and electrode terminal(s) 463 is constantly immersed in the fluid. The power supply 28 is then turned on and adjusted such that a high frequency voltage difference is applied between electrode terminals 462 and return electrode 624. The electrically conductive fluid provides the conduction path (see current flux lines) between electrode terminals 463 and the return electrode 624.

In the preferred embodiment, the high frequency voltage is sufficient to convert the electrically conductive fluid (not shown) between the occlusive media and electrode terminal(s) 463 into an ionized vapor layer or plasma. As a result of the applied voltage difference between electrode terminal(s) 463 and the occlusive media, charged particles in the plasma are accelerated towards the occlusion to cause dissociation of the molecular bonds within tissue structures, as discussed above. During the process, products of ablation and excess electrically conductive fluid, and other fluids (e.g., blood) may be aspirated from the target site to facilitate the surgeon's view. During ablation of the tissue, the residual heat generated by the current flux lines (typically less than 150°C), will usually be sufficient to coagulate any severed blood vessels at the site. If not, the surgeon may switch the power supply 28 into the coagulation mode by lowering the voltage to a level below the threshold for fluid vaporization, as discussed above. This simultaneous hemostasis results in less bleeding and facilitates the surgeon's ability to perform the procedure. Once the blockage has been removed, aeration and drainage are reestablished to allow the sinuses to heal and return to their normal function.

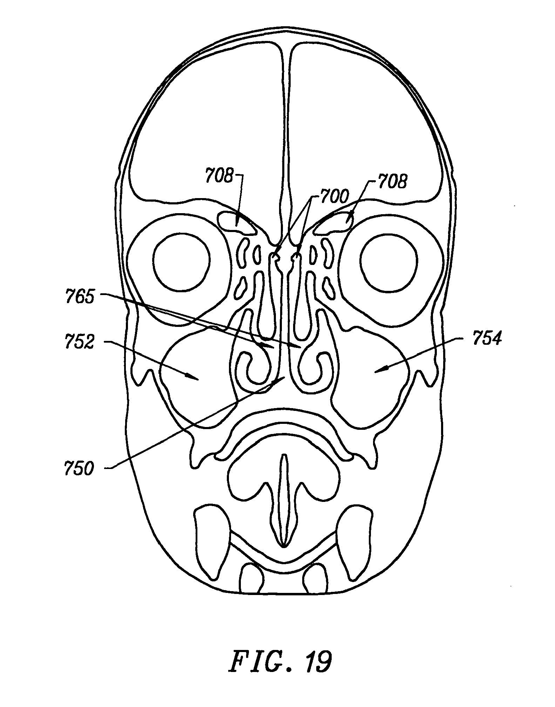

Fig. 19 is a coronal section of a patient's head illustrating the paranasal sinuses in more detail. As shown, the nasal septum 750 extends through the center of the nasal cavity 700 between the right and left portions 752, 754 of the maxillary sinus. A series of winding passages 765 connect the nasal cavity 700 with the maxillary sinus portions 752, 754. These passages 765 can become partially or completely blocked. The systems and methods of the present invention allow the surgeon to advance a small catheter into these passages and volumetrically remove the blockage in a minimally invasive manner, i.e., without causing significant damage to the surrounding cartilage, the nasal septum or the sinuses.

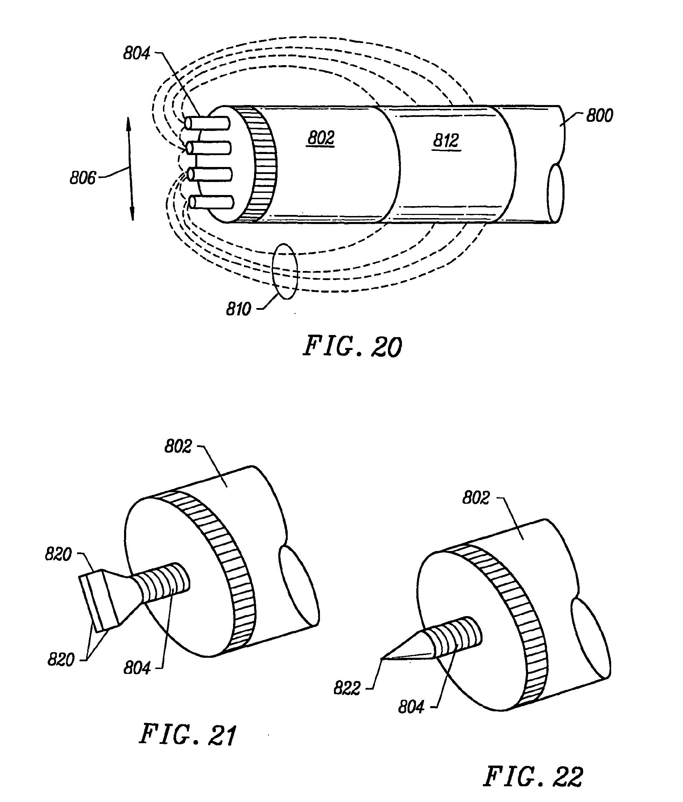

Fig. 20 illustrates an embodiment of the present invention designed for cutting of body structures. In this embodiment, the electrode terminals 804 are arranged in a linear or columnar array of one of more closely spaced columns so that as the electrodes 804 are moved along the longer axis (denoted by arrow 806 in Fig. 20), the current flux lines 810 are narrowly confined at the tip of the electrode terminals 804 and result in a cutting effect in the body structure being treated. As before, the current flux lines 810 emanating from the electrode terminals 804 pass through the electrically conducting liquid to the return electrode structure 812 located proximal to the probe tip.

Referring now to Figs. 21 and 22, alternative geometries are shown for the electrode terminals 804. These alternative electrode geometries allow the electrical current densities emanating from the electrode terminals 804 to be concentrated to achieve an increased ablation rate and/or a more concentrated ablation effect due to the fact that sharper edges (*i*.*e*., regions of smaller radii of curvature) result in higher current densities. Fig. 21 illustrates a flattened extension of a round wire electrode terminal 804 which results in higher current densities at the edges 820. Another example is shown in Fig. 22 in which the electrode terminal 804 is formed into a cone shaped point 822 resulting in higher current densities at the tip of the cone.

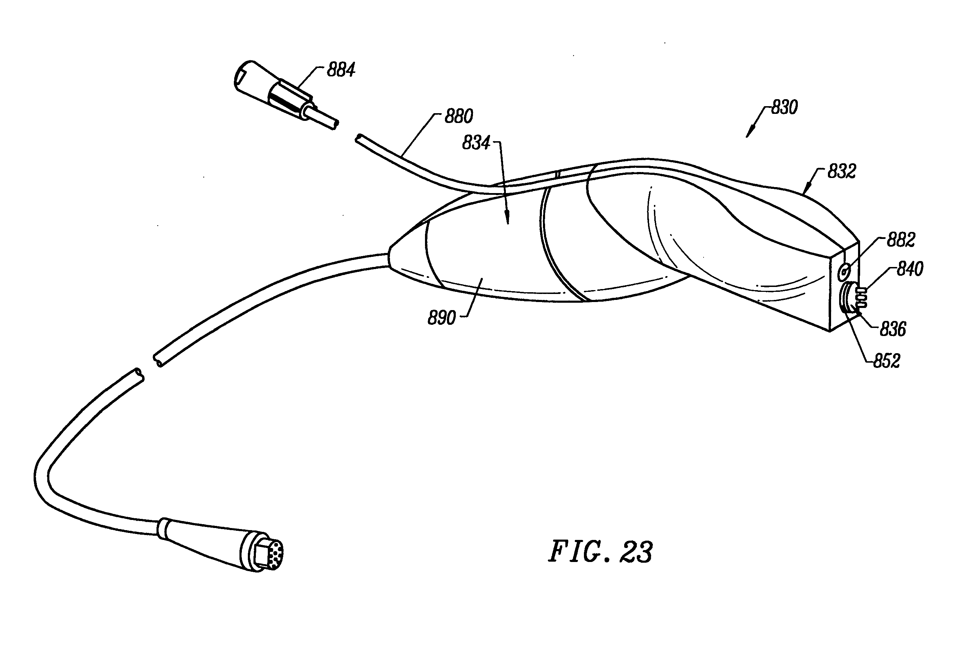

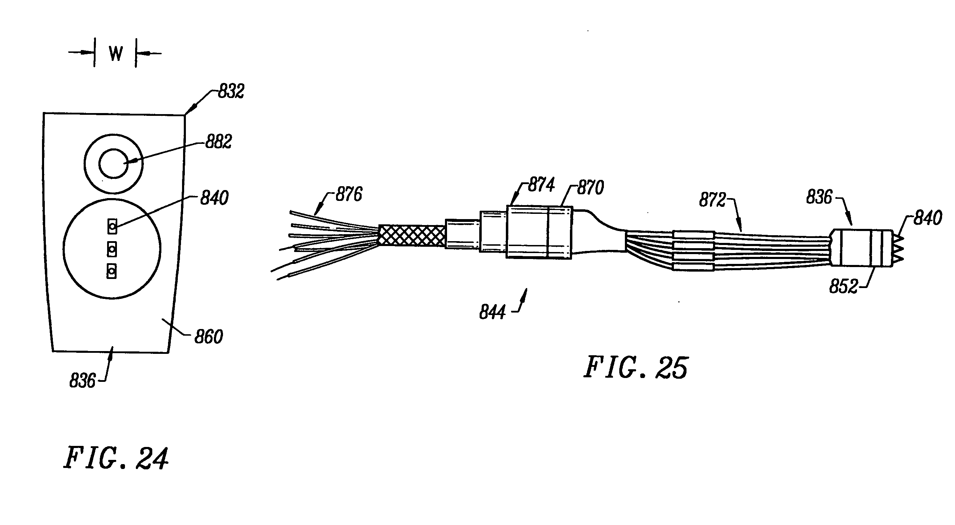

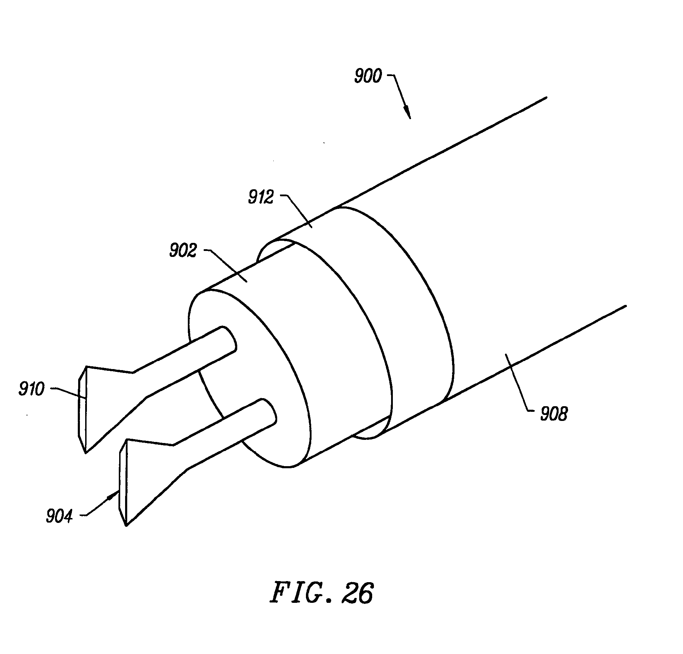

Figs. 23-25 illustrate an exemplary electrosurgical probe 830 or "Plasma Scalpel" for cutting and removing structures from the outer surface of the skin, such as lesions, scars, etc., or for cutting and removing tissue within the patient's nose, mouth and throat. Probe 830 comprises a shaft or disposable tip 832 removably coupled to a proximal handle 834, and an electrically insulating electrode support member 836 extending from tip 832 for supporting a plurality of electrode terminals 840 (see Figs. 23 and 25). Tip 832 and handle 834 typically comprise a plastic material that is easily molded into a suitable shape for handling by the surgeon. As shown in Fig. 26, handle 834 defines an inner cavity 842 that houses the electrical connections 844, and provides a suitable interface for connection to electrical connecting cable 34 (see Fig. 1). In the exemplary embodiment, handle 834 is constructed of a steam autoclavable plastic or metal (e.g., polyethylether keytone, or a stable metal alloy containing aluminum and/or zine. so that it can be re-used by sterilizing handle 834 between surgical procedures. High service temperature materials are preferred, such as a silicone cable jacket and a poly-ether-imide handpiece or ULTEM® that can withstand a repeated exposure to high temperatures.

Referring to Fig 23, tip 834 preferably comprises first and second housing halves that snap fit together, and form a recess therebetween for holding electrode support member 836 within the tip 832. Electrode support member 836 extends from the distal end of tip 832 (usually about 0.5 to 20 mm), and provides support for a plurality of electrically isolated electrode terminals 840 and one or more return electrodes 852 (see Fig. 25). Alternatively, electrode support member 836 may be recessed from the distal end of tip 832 to help confine the electrically conductive fluid around the electrode terminals 840 during the surgical procedure, as discussed above. Electrode support member 836 has a substantially planar tissue treatment surface 860 that is usually disposed at an angle of about 10 to 90 degrees relative to the longitudinal axis of handle 834 to facilitate handling by the surgeon. In the exemplary embodiment, this function is accomplished by orienting tip 832 at an acute angle relative to the longitudinal axis of handle 834.

In the embodiment shown in Figs. 23-25, probe 830 includes a single annular return electrode 452 for completing the current path between electrode terminals 840 and power supply 28 (see Fig. 1). As shown, return electrode 852 preferably has a fluid contact surface slightly proximal to tissue treatment surface 860, typically about 0.1 to 2 mm, preferably about 0.2 to 1 mm. Return electrode 852 is coupled to a connector (not shown) that extends to the proximal end of handle 834, where it is suitably connected to power supply 28 (Fig. 1).

Referring to Fig. 26, tip 832 further includes a male electrical connector 870 that holds a plurality of wires 872 each coupled to one of the electrode terminals 840 and the return electrode 852 on support member 836. A female connector 874 housed within handle 834 is removably coupled to male connector 870, and a plurality of wires 876 extend from female connector 874 to cable 34 (Fig. 1). Probe 830 will preferably also include an identification element, such as a coded resistor (not shown), for programming a particular voltage output range and mode of operation for the power supply. This allows the power supply to be employed with a variety of different probes for a variety of different applications.

In the representative embodiment, probe 830 includes a fluid tube 880 (Fig. 23) for delivering electrically conductive fluid to the target site. Fluid tube 880 is sized to extend through a groove in handle 834 and through an inner cavity in tip 832 to a distal opening 882 (Fig. 24) located adjacent electrode support member 836. Fluid tube 880 includes a proximal connector 4884for coupling to an electrically conductive fluid source 21 (Fig. 1).

Probe 830 will also include a valve or equivalent structure for controlling the flow rate of the electrically conducting fluid to the target site. In the representative embodiment, handle 312 comprises a rotatable sleeve 890 to provide a valve structure for fluid tube 880. Rotation of sleeve 890 will impede, and eventually obstruct, the flow of fluid through tube 880. Of course, this fluid control may be provided by a variety of other input and valve devices, such as switches, buttons, etc.

Referring to Figs. 24 and 25, electrically isolated electrode terminals 840 are spaced apart over tissue treatment surface 860 of electrode support member 836, preferably in a linear array. In the representative embodiment, three electrode terminals 840, each having a substantially conical shape, are arranged in a linear array extending distally from surface 860. Electrode terminals 840 will usually extend a distance of about 0.5 to 20 mm from tissue treatment surface 860, preferably about 1 to 5 mm. Applicant has found that this configuration increases the electric field intensities and associated current densities at the distal edges of electrode terminals 840, which increases the rate of tissue cutting. In the representative embodiment, the tissue treatment surface 380 has a circular cross-sectional shape with a diameter in the range of about 0.5 mm to 20 mm (preferably about 2 to 10 mm). The individual electrode terminals 840 preferably taper outward as shown, or they may form a distal edge, such as the electrodes shown in Figs. 8A and 11.

Electrode support member 836 preferably comprises a multilayer substrate comprising a suitable high temperature, electrically insulating material, such as ceramic. The multilayer substrate is a thin or thick-film hybrid having conductive strips that are adhered to the ceramic wafer layers (e.g., thick-film printed and fired onto or plated onto the ceramic wafers). The conductive strips typically comprise tungsten, gold, nickel, silver, platinum or equivalent materials. In the exemplary embodiment, the conductive strips comprise tungsten, and they are co-fired together with the wafer layers to form an integral package. The conductive strips are coupled to external wire connectors by holes or vias that are drilled through the ceramic layers, and plated or otherwise covered with conductive material. A more complete description of such support members 370 can be found in U.S. patent application no. 08/977,845, filed November 25, 1997 (Attorney Docket No. D-2),

Referring now to Figs. 26, another embodiment of an electrosurgical probe 900 comprises a shaft 908 and at least two electrode terminals 904 extending from a support matrix 902 at the distal end of the shaft. The electrode terminals 904 preferably define a distal edge 910 for cutting an incision in tissue. The edges 910 of the electrode terminals 904 are substantially parallel with each other and usually spaced a distance of about 4 to 15 mm, preferably about 8-10 mm. The edges 910 extend from the distal end of support matrix 902 by a distance of about 0.5 to 10 mm, preferably about 2 to 5 mm. In the exemplary embodiment, probe 900 will include a return electrode 912 spaced proximally from the electrode terminals 904. Alternatively, the return electrode 912 may be one of the electrode terminals 904, or it may be a dispersive pad located on an external surface of the patient's body.

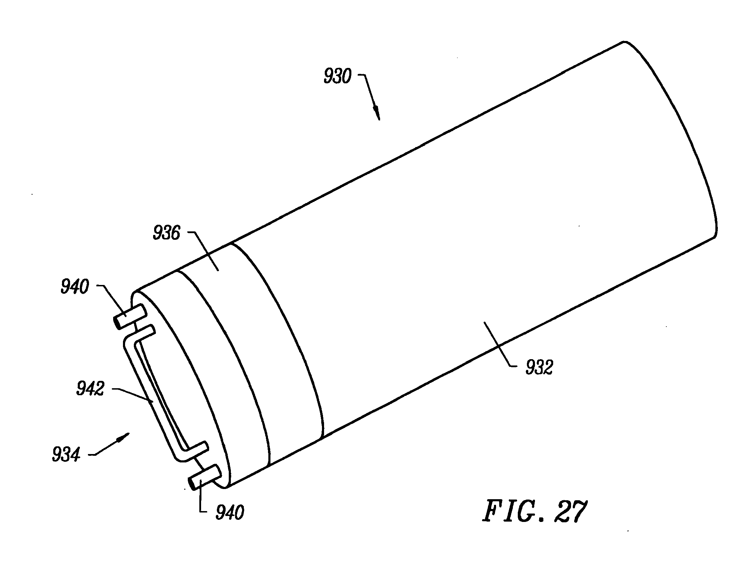

Referring to Fig. 27, an electrosurgical scalpel 930 comprises a shaft 932 with an electrode assembly 934 at its distal end, and a return electrode 936 proximally spaced from electrode assembly 934 by an insulating member 938 (similar to some of the devices described above). In this embodiment, electrode assembly 934 comprises a pair of outer electrode terminals 940 and an inner loop electrode 942 aligned with each other to form a substantially linear cutting path for cutting through tissue. The electrodes in this embodiment will generally have the same extension lengths and sizes as described above. This embodiment is particularly useful for cutting tissue and effecting simultaneous hemostasis of the cut tissue. The outer electrode terminals 940 extend distally to a distinct, small area or point for precise cutting of tissue, while the loop electrode 942 provides sufficient exposed surface area to effectively coagulate tissue. In severely bleeding tissue, the power supply 28 will be switched into the subablation mode to remove the plasma layer and increase the effectiveness of coagulation. Alternatively, the surgeon may decrease or eliminate the supply of electrically conductive fluid to the target site and remain at a higher power level. Applicant has found that this forces the blood to become the conductive path between the return electrode 936 and the electrode terminals 940, which increases the rate of coagulation in the target area.

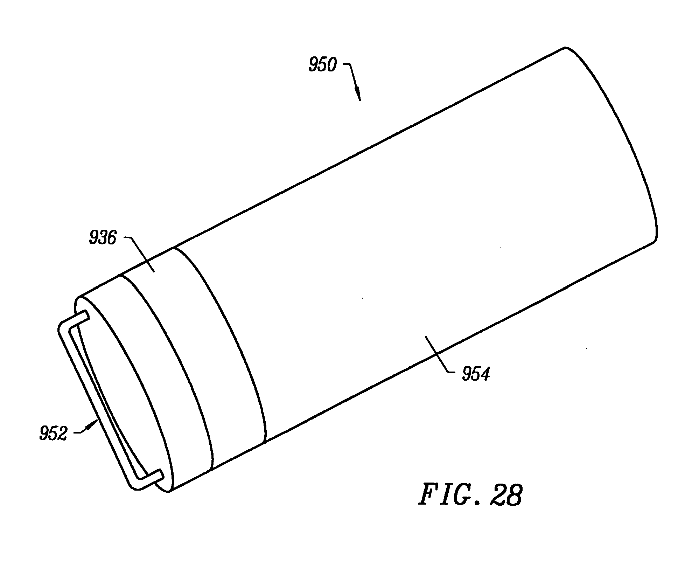

Fig. 28 illustrates yet another electrosurgical scalpel 950 according to the present invention. Scalpel 950 comprises a single loop electrode 952 at the distal end of the instrument shaft 954 for both cutting and coagulation of tissue. Similar to the above embodiment, the sharp distal line formed by the loop electrode 952 provides efficient and precise cutting of tissue, while the increased surface area of the loop electrode facilitates hemostasis. In other embodiments, the scalpel 950 may include a plurality of such loops, aligned linearly, in parallel or in an array.

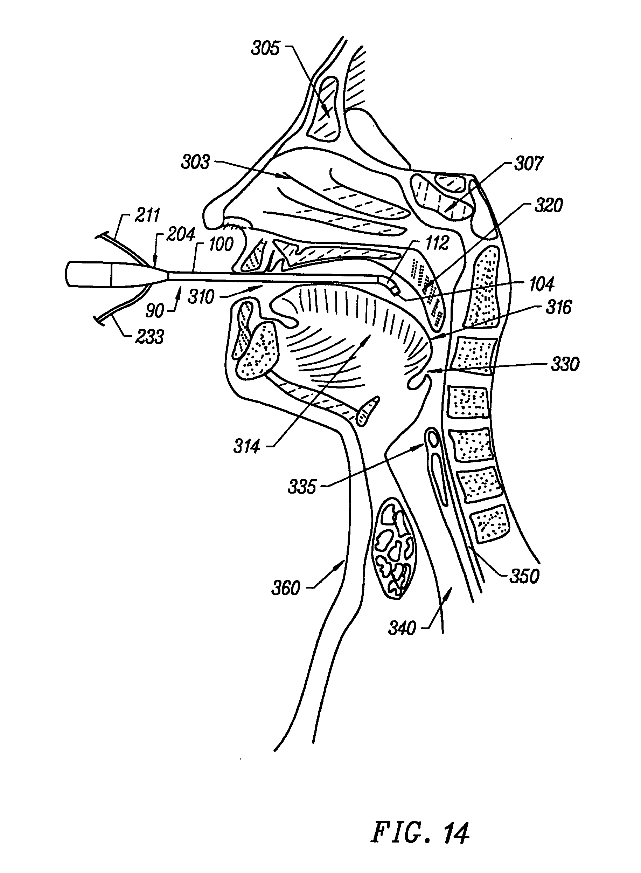

Methods for treating air passage disorders according to the present invention will now be described. In these embodiments, an electrosurgical probe such as one of those described above can be used to ablate, cut or resect targeted masses including, but not limited to, the tongue, tonsils, turbinates, adenoids, soft palate tissues (e.g., the uvula and soft palate), hard tissue and other mucosal or submucosal tissue. In one embodiment, selected portions of the uvula 320, soft palate and tonsils are removed to treat sleep apnea. In this method, the distal end of an electrosurgical instrument 90 such as one of the probes or catheters discussed above is introduced into the patient's mouth 310, as shown in Fig. 14. This procedure is typically accomplished in a hospital under general anesthesia, although it is possible to perform the procedure in an office setting under local anesthesia, e.g., with a tumescent or other local anesthesia. An endoscope (not shown), or other type of viewing device, may also be introduced, or partially introduced, into the mouth 310 to allow the surgeon to view the procedure (the viewing device may be integral with, or separate from, the electrosurgical probe). The electrode terminals 104 are positioned adjacent to or against the target tissue (e.g., tonsils, uvula 320, soft palate, etc.) and electrically conductive fluid is delivered to the target site as described above. The power supply 28 (Fig. 1) is then activated and a high frequency voltage difference is applied between the electrode terminals 104 and return electrode 112. Depending on the procedure and the configuration of the distal end of probe 90, the surgeon will then manipulate the probe 90 to cut, ablate or otherwise remove the obstructive tissue without damaging sensitive structures, such as nerves and non-target tissue in the mouth. The target site may also be aspirated to improve visualization and to ensure that excess fluid and/or products of ablation do not flow down the patient's throat.

As discussed above, the present invention uses a novel Coblation process for removing tissue that involves lower temperatures than conventional RF and laser devices used for treating sleep obstructive disorders. Accordingly, the tissue is removed with minimal charring and extremely low depths of tissue necrosis. This minimal collateral damage and low temperature results in significantly less pain to the patient during and after the operation, and increased healing times over conventional devices. In addition, the low temperature plasma at the end of the probe 90 provides simultaneous hemostasis of any severed blood vessels in the region of the target tissue. This minimizes bleeding during the operation, which increases visualization, decreases the duration of the operation and possibly contributes to faster healing and less post-operative pain. Moreover, the precise nature of the Coblation mechanism provides some comfort to the surgeon that damage to adjacent nerves and other sensitive structures will be minimized or completely eliminated.

In some cases, patient bleeding may be more intense than in others. In particular, the removal of infected tonsils often presents a significant hemostasis problem to the surgeon. Applicant has found that the electrosurgical probes described in Figs. 27 and 28 are particularly well suited for coagulating and sealing severed blood vessels in the tonsils during the ablation procedure. In severe cases, applicant has found that eliminating the presence of conductive fluid (e.g., saline) at the target site facilitates coagulation and hemostasis (e.g., by turning the pump off and entering the coagulation mode).

In another embodiment, the electrosurgical probe of the present invention can be used to ablate and/or contract soft palate tissue to treat snoring disorders. In particular, the probe is used to ablate or shrink sections of the uvula 320 without causing unwanted tissue damage under and around the selected sections of tissue. For tissue contraction, a sufficient voltage difference is applied between the electrode terminals 104 and the return electrode 112 to elevate the uvula tissue temperature from normal body temperatures (e.g., 37°C) to temperatures in the range of 45°C to 90°C, preferably in the range from 60°C to 70°C. This temperature elevation causes contraction of the collagen connective fibers within the uvula tissue.

In one method of tissue contraction according to the present invention, an electrically conductive fluid is delivered to the target site as described above, and heated to a sufficient temperature to induce contraction or shrinkage of the collagen fibers in the target tissue. The electrically conducting fluid is heated to a temperature sufficient to substantially irreversibly contract the collagen fibers, which generally requires a tissue temperature in the range of about 45°C to 90°C, usually about 60°C to 70°C. The fluid is heated by applying high frequency electrical energy to the electrode terminal(s) in contact with the electrically conducting fluid. The current emanating from the electrode terminal(s) 104 heats the fluid and generates a jet or plume of heated fluid, which is directed towards the target tissue. The heated fluid elevates the temperature of the collagen sufficiently to cause hydrothermal shrinkage of the collagen fibers. The return electrode 112 draws the electric current away from the tissue site to limit the depth of penetration of the current into the tissue, thereby inhibiting molecular dissociation and breakdown of the collagen tissue and minimizing or completely avoiding damage to surrounding and underlying tissue structures beyond the target tissue site. In an exemplary embodiment, the electrode terminal(s) 104 are held away from the tissue a sufficient distance such that the RF current does not pass into the tissue at all, but rather passes through the electrically conducting fluid back to the return electrode. In this embodiment, the primary mechanism for imparting energy to the tissue is the heated fluid, rather than the electric current.

In an alternative embodiment, the electrode terminal(s) 104 are brought into contact with, or close proximity to, the target tissue so that the electric current passes directly into the tissue to a selected depth. In this embodiment, the return electrode draws the electric current away from the tissue site to limit its depth of penetration into the tissue. Applicant has discovered that the depth of current penetration also can be varied with the electrosurgical system of the present invention by changing the frequency of the voltage applied to the electrode terminal and the return electrode. This is because the electrical impedance of tissue is known to decrease with increasing frequency due to the electrical properties of cell membranes which surround electrically conductive cellular fluid. At lower frequencies (e.g., less than 350 kHz), the higher tissue impedance, the presence of the return electrode and the electrode terminal configuration of the present invention (discussed in detail below) cause the current flux lines to penetrate less deeply resulting in a smaller depth of tissue heating. In an exemplary embodiment, an operating frequency of about 100 to 200 kHz is applied to the electrode terminal(s) to obtain shallow depths of collagen shrinkage (e.g., usually less than 1.5 mm and preferably less than 0.5 mm).

In another aspect of the invention, the size (e.g., diameter or principal dimension) of the electrode terminals employed for treating the tissue are selected according to the intended depth of tissue treatment. As described previously in copending patent application PCT International Application, U.S. National Phase Serial No. PCT/US94/05168, the depth of current penetration into tissue increases with increasing dimensions of an individual active electrode (assuming other factors remain constant, such as the frequency of the electric current, the return electrode configuration, etc.). The depth of current penetration (which refers to the depth at which the current density is sufficient to effect a change in the tissue, such as collagen shrinkage, irreversible necrosis, etc.) is on the order of the active electrode diameter for the bipolar configuration of the present invention and operating at a frequency of about 100kHz to about 200kHz. Accordingly, for applications requiring a smaller depth of current penetration, one or more electrode terminals of smaller dimensions would be selected. Conversely, for applications requiring a greater depth of current penetration, one or more electrode terminals of larger dimensions would be selected.

In addition to the above procedures, the system and method of the present invention may be used for treating a variety of disorders in the mouth 310, pharynx 330, larynx 335, hypopharynx, trachea 340, esophagus 350 and the neck 360. For example, tonsillar hyperplasis or other tonsil disorders may be treated with a tonsillectomy by partially ablating the lymphoepithelial tissue. This procedure is usually carried out under intubation anesthesia with the head extended. An incision is made in the anterior faucial pillar, and the connective tissue layer between the tonsillar parenchyma and the pharyngeal constrictor muscles is demonstrated. The incision may be made with conventional scalpels, or with the electrosurgical probe of the present invention. The tonsil is then freed by ablating through the upper pole to the base of the tongue, preserving the faucial pillars. The probe ablates the tissue, while providing simultaneous hemostasis of severed blood vessels in the region. Similarly, adenoid hyperplasis, or nasal obstruction leading to mouth breathing difficulty, can be treated in an adenoidectomy by separating (e.g., resecting or ablating) the adenoid from the base of the nasopharynx.

Other pharyngeal disorders can be treated according to the present invention. For example, hypopharyngeal diverticulum involves small pouches that form within the esophagus immediately above the esophageal opening. The sac of the pouch may be removed endoscopically according to the present invention by introducing a rigid esophagoscope, and isolating the sac of the pouch. The cricopharyngeus muscle is then divided, and the pouch is ablated according to the present invention. Tumors within the mouth and pharynx, such as hemangionmas, lymphangiomas, papillomas, lingual thyroid tumors, or malignant tumors, may also be removed according to the present invention.

Other procedures of the present invention include removal of vocal cord polyps and lesions and partial or total laryngectomies. In the latter procedure, the entire larynx is removed from the base of the tongue to the trachea, if necessary with removal of parts of the tongue, the pharynx, the trachea and the thyroid gland.

Tracheal stenosis may also be treated according to the present invention. Acute and chronic stenoses within the wall of the trachea may cause coughing, cyanosis and choking.

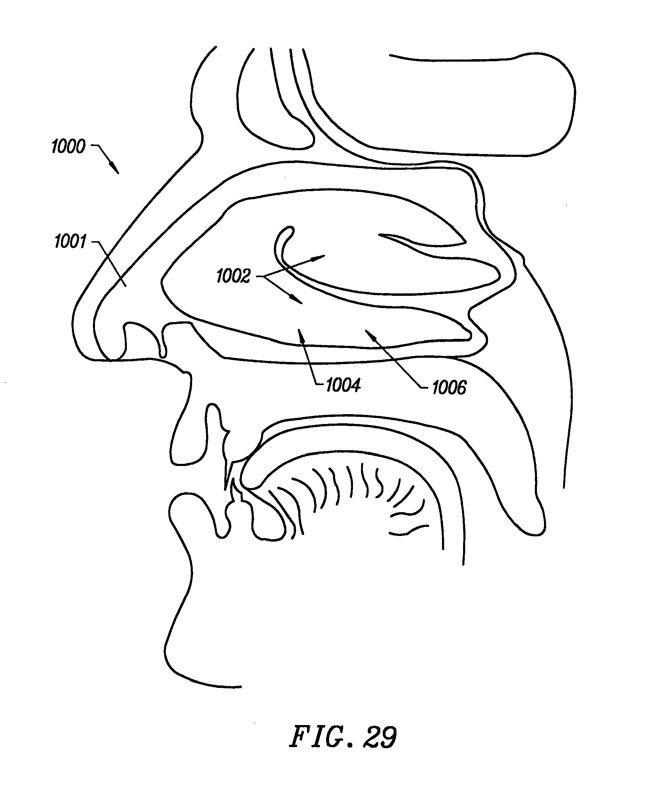

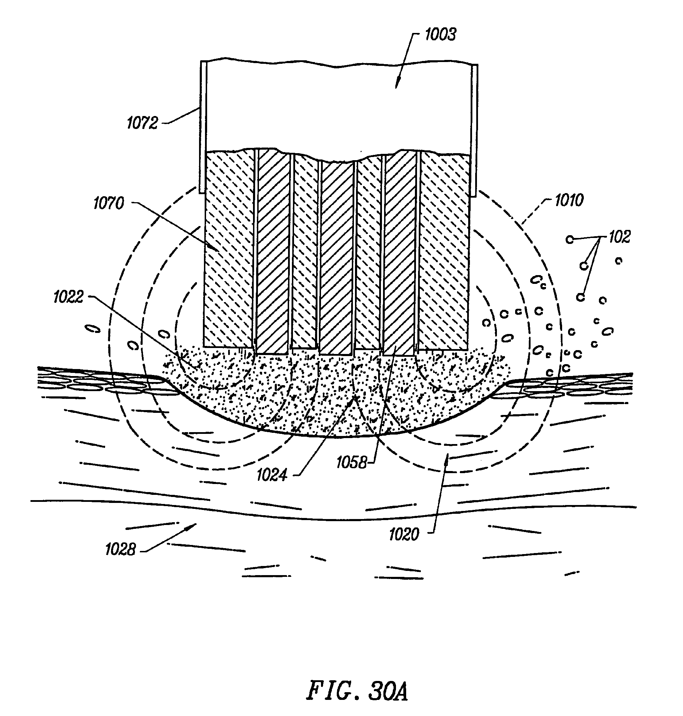

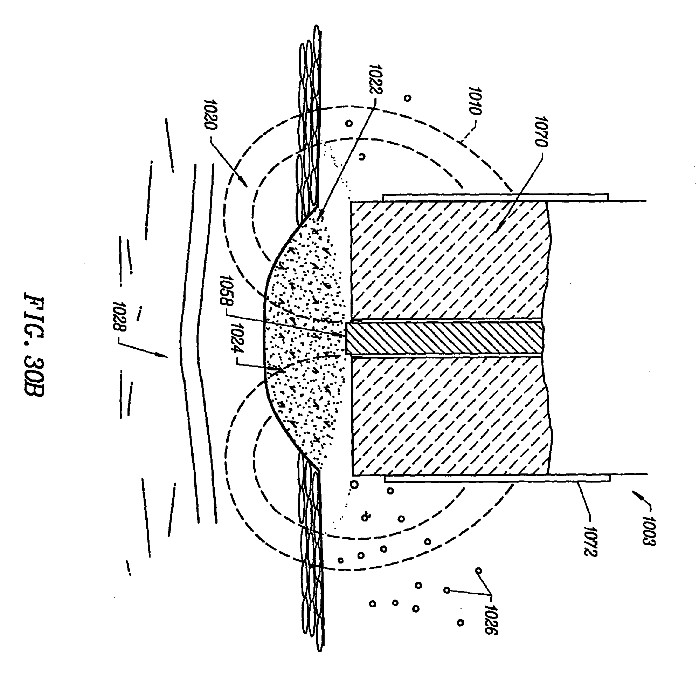

Figs. 29 and 30 illustrate a method for treating enlarged body structures, such as polyps or turbinates, according to the present invention. In these procedures, the polyps, turbinates or other sinus tissue may be ablated or reduced (e.g., by tissue contraction) to clear the blockage and/or prevent further swelling of the turbinates to reestablish normal sinus function. For example, in chronic rhinitis, which is a collective term for chronic irritation or inflammation of the nasal mucosa with hypertrophy of the nasal mucosa, the inferior turbinate may be reduced by ablation or contraction. Alternatively, a turbinectomy or mucotomy may be performed by removing a strip of tissue from the lower edge of the inferior turbinate to reduce the volume of the turbinate. For treating nasal polypi, which comprises benign pedicled or sessile masses of nasal or sinus mucosa caused by inflammation, the nasal polypi may be contracted or shrunk, or ablated by the method of the present invention. For treating severe sinusitis, a frontal sinus operation may be performed to introduce the electrosurgical probe to the site of blockage. The present invention may also be used to treat diseases of the septum, e.g., ablating or resecting portions of the septum for removal, straightening or reimplantation of the septum.

The present invention is particularly useful in reducing enlarged turbinates by volumetrically removing a portion of the turbinates. As shown in Fig. 29, a patient's nose 1000 comprises a nasal cavity 1001 having a set of turbinates 1002, including a middle nasal concha 1004 and an inferior nasal concha 1006. The inferior nasal concha 1006 generally comprises an anterior portion and a posterior portion. It has been found that ablating the inferior nasal concha 1006, typically the anterior portion, does not substantially degrade its function. According to the present invention, the distal end of a probe 1003 (Fig. 30A) is introduced through a nasal passage into the nasal cavity 1001. The electrode terminals 1058 are positioned adjacent the selected turbinate 1006 and electrically conductive fluid is delivered through a fluid delivery element (not shown) to the tissue. The fluid flows past the return electrode 1072 to the electrode terminals 1058 at the distal end of the shaft. The rate of fluid flow may be controlled with a valve (not shown) such that the zone between the tissue and electrode support 1070 is constantly immersed in the fluid. Alternatively, or in conjunction with fluid delivery, an electrically conductive gel may be applied to the target site prior to the procedure. The power supply 28 is then turned on and adjusted such that a high frequency voltage difference is applied between electrode terminals 1058 and return electrode 1072. The electrically conductive fluid provides the conduction path between electrode terminals 1058 and the return electrode 1072. Once the probe 1003 has been activated, the surgeon will positioned the electrode terminals in contact with, or close proximity to, the turbinate 1006 to volumetrically remove turbinate tissue.

Figs. 30A and 30B illustrate the removal of sinus tissue in more detail (Fig. 30B illustrates a single active electrode embodiment). As shown, a high frequency voltage difference is applied between electrode terminal(s) 1058 and return electrode 1072 such that electric current 1010 flows through the conductive fluid to the high frequency voltage is sufficient to convert the electrically conductive fluid (not shown) between the target tissue 1020 and electrode terminal(s) 1058 into an ionized vapor layer 1022 or plasma. As a result of the applied voltage difference between electrode terminal(s) 1058 and the target tissue 1020 (i.e., the voltage gradient across the plasma layer 1022), charged particles 1024 in the plasma (viz., electrons) are accelerated towards the tissue. At sufficiently high voltage differences, these charged particles 1024 gain sufficient energy to cause dissociation of the molecular bonds within tissue structures in contact with the plasma layer 1022. This molecular dissociation is accompanied by the volumetric removal (i.e., ablative sublimation) of tissue and the production of low molecular weight gases 1026, such as oxygen, nitrogen, carbon dioxide, hydrogen and methane. The short range of the accelerated charged particles 1024 within the tissue confines the molecular dissociation process to the surface layer to minimize damage and necrosis to the underlying tissue 1028.

During the process, the gases 1026 may be aspirated through a suction tube, instrument or lumen with probe 1003 (not shown) suitably coupled to a vacuum source. In addition, excess electrically conductive fluid, and other fluids (e.g., blood) may be aspirated from the target site to facilitate the surgeon's view. During ablation of the tissue, the residual heat generated by the current flux lines 1010 (typically less than 150°C), will usually be sufficient to coagulate any severed blood vessels at the site. If not, the surgeon may switch the power supply 28 into the coagulation mode by lowering the voltage to a level below the threshold for fluid vaporization, as discussed above. This simultaneous hemostasis results in less bleeding and facilitates the surgeon's ability to perform the procedure. Once the turbinate has been reduced, aeration and drainage are reestablished to allow the sinuses to heal and return to their normal function.

Depending on the procedure, the surgeon may translate the electrode terminals 1058 relative to the turbinate tissue to form holes, channels, stripes, divots, craters or the like within the turbinate. In addition, the surgeon may purposely create some thermal damage within these holes, or channels to form scar tissue that will inhibit the turbinate from swelling after the procedure. In one embodiment, the physician axially translates the electrode terminals 1058 into the turbinate tissue as the tissue is volumetrically removed to form one or more holes in the turbinate, typically having a diameter of less than 2 mm, preferably less than 1 mm. In another embodiment, the physician translates the electrode terminals 1058 across the outer surface of the turbinates to form one or more channels or troughs. Applicant has found that the present invention can quickly and cleanly create such holes, divots or channels in tissue with the cold ablation technology described herein. A more complete description of methods for forming holes or channels in tissue can be found in U.S. Patent No. 5,683,366, the complete disclosure of which is incorporated herein by reference for all purposes.

Another advantage of the present invention is the ability to precisely ablate channels or holes within the turbinates without causing necrosis or thermal damage to the underlying and surrounding tissues, nerves (e.g., the optic nerve) or bone. In addition, the voltage can be controlled so that the energy directed to the target site is insufficient to ablate bone or adipose tissue (which generally has a higher impedance than the target sinus tissue). In this manner, the surgeon can literally clean the tissue off the bone, without ablating or otherwise effecting significant damage to the bone.

## Claims

1. An apparatus for applying electrical energy to a target site within or on a patient's body, the apparatus comprising:
an instrument having a shaft (18) with a proximal end portion and a distal end portion and an electrode terminal (58) disposed near the distal end portion of the shaft;
a return electrode (112);
a high frequency power supply (28) coupled to the electrode terminal (58) and the return electrode (112) for applying therebetween a voltage difference sufficient to volumetrically remove tissue from the target site; and
fluid supply means having a fluid outlet for delivering electrically conducting fluid to the target site, wherein the electrode terminal and the fluid outlet are recessed from an outer surface of the apparatus to confine the electrically conducting fluid to a region surrounding the electrode terminal.

2. The apparatus of Claim 1 wherein the shaft is shaped to form a cavity around the electrode terminal and the fluid outlet.

3. The apparatus of any preceding claim wherein the electrode terminal comprises a plurality of active electrodes held in an electrode support.

4. The apparatus of any preceding claim wherein the apparatus comprises a nasal surgery apparatus for applying electrical energy within a nasal cavity or sinus of a patient's nose.

5. The apparatus of any preceding claim wherein the shaft is sized to enable the distal end portion of the shaft to advance into a nasal cavity or sinus to reach the target site within that nasal cavity or sinus.

6. The apparatus of any preceding claim wherein the voltage is arranged to create an electric field to cause vaporization of the electrically conducting fluid at the target site to create an ionised fluid plasma.

7. The apparatus of any preceding claim wherein the tissue is removed without causing necrosis or thermal damage to surrounding tissue.

8. The apparatus of any preceding claim wherein the distal end portion of the shaft (18) has a diameter less than 3mm.

9. The apparatus of any preceding claim wherein the distal end portion of the shaft (18) has a diameter less than 1mm.

10. The apparatus of any preceding claim wherein the return electrode forms a portion of the shaft.

11. The apparatus of any preceding claim, further including an insulating member (938) positioned between the return electrode (112, 936) and the electrode terminal (58, 934), the insulating member (938) spacing the return electrode from the electrode terminal to minimize direct contact between the return electrode and a body structure at the target site when the electrode terminal is positioned in close proximity or in partial contact with the body structure.

12. The apparatus of any preceding claim, wherein the distal end portion of the shaft is configured for axial translation into a channel formed by the volumetric removal of tissue.

13. The apparatus of Claim 12, further comprising means for thermally damaging the inner walls of the channel formed by the volumetric removal of tissue without removing or ablating said inner walls.

14. The apparatus of Claim 13, wherein the return electrode (112) is sized and positioned relative to the electrode terminal (58) such that, when the return electrode has been axially translated into the channel, the voltage difference supplied by the power supply (28) will cause current to flow from the electrode terminal (58), through the inner walls of the channel to the return electrode (112), such that the current causes thermal damage to the inner walls of the channel.

15. The apparatus of any of the preceding claims, wherein the shaft is rigid.

16. The apparatus of any of the preceding claims, wherein the shaft has a bend to allow the distal end portion to be offset relative to the proximal end portion.

17. The apparatus of any of the preceding claims, wherein the electrode terminal (104) is carried by a support member at an angle of 10 to 90 degrees to the longitudinal axis of the shaft.

18. The apparatus of any of the preceding claims, further comprising an aspiration element for aspirating fluid from the target site.

19. The apparatus of any of the preceding claims, wherein the shaft has a fluid delivery element (14, 15) for supplying fluid to the target site, the fluid delivery element defining a fluid path in electrical contact with the return electrode (112) and the electrode terminal (58).

20. The apparatus of any preceding claim, wherein the return electrode (112) is a tubular member and the fluid delivery element comprises an axial lumen (54) coupled to the return electrode, the axial lumen forming at least a portion of the fluid path and having an outlet in fluid communication with the electrode terminal (58).

21. The apparatus of any preceding claim, wherein the target site comprises a turbinate within a nasal cavity of a patient's nose.

## Patentansprüche

1. Vorrichtung zum Einbringen elektrischer Energie in einen Zielort in oder an einem Patientenkörper, wobei die Vorrichtung umfasst:
ein Instrument mit einem Schaft (18), der einen proximalen Endabschnitt und einen distalen Endabschnitt sowie einen Elektrodenanschluss (48), der in der Nähe des distalen Endabschnitts des Schafts angeordnet ist, aufweist;
eine Rückleitungselektrode (112);
eine Hochfrequenz-Stromversorgung (28), die mit dem Elektrodenanschluss (58) und mit der Rückleitungselektrode (112) gekoppelt ist, um zwischen ihnen eine Spannungsdifferenz anzulegen, die ausreicht, um Gewebe von dem Zielort volumetrisch zu entfernen; und
Fluidversorgungsmittel, die einen Fluidauslass besitzen, um elektrisch leitendes Fluid an den Zielort abzugeben, wobei der Elektrodenanschluss und der Fluidauslass aus einer äußeren Oberfläche der Vorrichtung ausgenommen sind, um elektrisch leitendes Fluid in einen den Elektrodenanschluss umgebenden Bereich einzuschließen.

2. Vorrichtung nach Anspruch 1, wobei der Schaft so geformt ist, dass er um den Elektrodenanschluss und den Fluidauslass einen Hohlraum bildet.

3. Vorrichtung nach einem vorhergehenden Anspruch, wobei der Elektrodenanschluss mehrere aktive Elektroden, die in einem Elektrodenträger gehalten werden, umfasst.

4. Vorrichtung nach einem vorhergehenden Anspruch, wobei die Vorrichtung eine nasenchirurgische Vorrichtung umfasst, um elektrische Energie in einen Nasenhohlraum oder einen Sinus einer Patientennase einzubringen.

5. Vorrichtung nach einem vorhergehenden Anspruch, wobei der Schaft so bemessen ist, dass sich der distale Endabschnitt des Schafts in einen Nasenhohlraum oder Sinus vorwärtsbewegen kann, um den Zielort in dem Nasenhohlraum oder Sinus zu erreichen.

6. Vorrichtung nach einem vorhergehenden Anspruch, wobei die Spannung so bemessen ist, dass sie ein elektrisches Feld erzeugt, das eine Verdampfung des elektrisch leitenden Fluids am Zielort hervorruft, um ein ionisiertes Fluidplasma zu erzeugen.

7. Vorrichtung nach einem vorhergehenden Anspruch, wobei das Gewebe ohne Verursachung einer Nekrose oder einer thermischen Beschädigung des umgebenden Gewebes entfernt wird.

8. Vorrichtung nach einem vorhergehenden Anspruch, wobei der distale Endabschnitt des Schafts (18) einen Durchmesser von weniger als 3 mm hat.

9. Vorrichtung nach einem vorhergehenden Anspruch, wobei der distale Endabschnitt des Schafts (18) einen Durchmesser von weniger als 1 mm hat.

10. Vorrichtung nach einem vorhergehenden Anspruch, wobei die Rückleitungselektrode einen Abschnitt des Schafts bildet.

11. Vorrichtung nach einem vorhergehenden Anspruch, die ferner ein isolierendes Element (938) umfasst, das zwischen der Rückleitungselektrode (112, 936) und dem Elektrodenanschluss (58, 934) positioniert ist, wobei das isolierende Element (938) die Rückleitungselektrode von dem Elektrodenanschluss beabstandet, um einen direkten Kontakt zwischen der Rückleitungselektrode und einer Körperstruktur am Zielort minimal zu machen, wenn der Elektrodenanschluss sehr nahe bei oder in einem teilweisen Kontakt mit der Körperstruktur positioniert ist.

12. Vorrichtung nach einem vorhergehenden Anspruch, wobei der distale Endabschnitt des Schafts für eine axiale translatorische Bewegung in einem durch die volumetrische Entfernung von dem Gewebe gebildeten Kanal konfiguriert ist.

13. Vorrichtung nach Anspruch 12, die ferner Mittel zum thermischen Beschädigen der Innenwände des durch die volumetrische Entfernung von Gewebe gebildeten Kanals ohne Entfernen oder Abtragen der innenwände umfasst.

14. Vorrichtung nach Anspruch 13, wobei die Rückleitungselektrode (112) so bemessen und relativ zu dem Elektrodenanschluss (58) positioniert ist, dass dann, wenn die Rückleitungselektrode axial translatorisch in den Kanal bewegt worden ist, die durch die Stromversorgung (28) gelieferte Spannungsdifferenz bewirkt, dass ein Strom von dem Elektrodenanschluss (58) durch die Innenwände des Kanals zu der Rückleitungselektrode (112) fließt, so dass der Strom eine thermische Beschädigung der Innenwände des Kanals hervorruft.

15. Vorrichtung nach einem der vorhergehenden Anspruche, wobei der Schaft starr ist.

16. Vorrichtung nach einem der vorhergehenden Ansprüche, wobei der Schaft eine Biegung besitzt, die ermöglicht, dass der distale Endabschnitt in Bezug auf den proximalen Endabschnitt versetzt ist.

17. Vorrichtung nach einem der vorhergehenden Ansprüche, wobei der Elektrodenanschluss (104) durch ein Trägerelement unter einem Winkel von 10 bis 90 Grad in Bezug auf die Längachse des Schafts getragen wird.

18. Vorrichtung nach einem der vorhergehenden Ansprüche, die ferner ein Saugelement zum Absaugen von Fluid von dem Zielort umfasst.

19. Vorrichtung nach einem der vorhergehenden Ansprüche, wobei der Schaft ein Fluidabgabeelement (14, 15) besitzt, um Fluid zu dem Zielort zu liefern, wobei das Fluidabgabeelement einen Fluidweg definiert, der in elektrischem Kontakt mit der Rückleitungselektrode (112) und dem Elektrodenanschluss (58) ist.

20. Vorrichtung nach einem vorhergehenden Anspruch, wobei die Rückleitungselektrode (112) ein röhrenförmige Element ist und das Fluidabgabeelement einen axialen Hohlraum (54) aufweist, der mit der Rückleitungselektrode gekoppelt ist, wobei der axiale Hohlraum wenigstens einen Abschnitt des Fluidwegs bildet und einen Auslass besitzt, der in einer Fluidkommunikation mit dem Elektrodenanschluss (58) seht.

21. Vorrichtung nach einem vorhergehenden Anspruch, wobei der Zielort eine Nasenmuschel in einem Nasenhohlraum einer Patientennase umfasst.

## Revendications

1. Appareil pour appliquer de l'énergie électrique à un site cible à l'intérieur du ou sur le corps d'un patient, l'appareil comprenant :
un instrument comportant un arbre (18) avec une portion d'extrémité proximale et une portion d'extrémité distale et une borne d'électrode (58) disposée près de la portion d'extrémité distale de l'arbre ;
une électrode de retour (112) ;
une alimentation électrique haute fréquence (28) couplée à la borne d'électrode (58) et à l'électrode de retour (112) pour appliquer entre elles une différence de tension suffisante pour enlever volumétriquement un tissu du site cible ; et
un moyen d'alimentation en fluide ayant une évacuation de fluide pour délivrer un fluide électriquement conducteur au site cible, dans lequel la borne d'électrode et l'évacuation de fluide sont évidées depuis une surface externe de l'appareil pour confiner le fluide électriquement conducteur à une région entourant la borne d'électrode.

2. Appareil selon la revendication 1, dans lequel l'arbre est façonné pour former une cavité autour de la borne d'électrode et de l'évacuation de fluide.

3. Appareil selon l'une quelconque des revendications précédentes, dans lequel la borne d'électrode comprend une pluralité d'électrodes actives maintenues dans un support d'électrode.

4. Appareil selon l'une quelconque des revendications précédentes, dans lequel l'appareil comprend un appareil de chirurgie nasale destiné à appliquer de l'énergie électrique à l'intérieur d'une cavité nasale ou d'un sinus du nez d'un patient.

5. Appareil selon l'une quelconque des revendications précédentes, dans lequel l'arbre est dimensionné pour permettre à la portion d'extrémité distale de l'arbre d'avancer dans une cavité nasale ou un sinus pour atteindre le site cible à l'intérieur de la cavité nasale ou du sinus.

6. Appareil selon l'une quelconque des revendications précédentes, dans lequel la tension est disposée pour créer un champ électrique pour provoquer une vaporisation du fluide électriquement conducteur au niveau du site cible pour créer un plasma de fluide ionisé.

7. Appareil selon l'une quelconque des revendications précédentes, dans lequel le tissu est enlevé sans entraîner de nécrose ou d'endommagement thermique du tissu alentour.

8. Appareil selon l'une quelconque des revendications précédentes, dans lequel la portion d'extrémité distale de l'arbre (18) a un diamètre inférieur à 3 mm.

9. Appareil selon l'une quelconque des revendications précédentes, dans lequel la portion d'extrémité distale de l'arbre (18) a un diamètre inférieur à 1 mm.

10. Appareil selon l'une quelconque des revendications précédentes, dans lequel l'électrode de retour forme une portion de l'arbre.

11. Appareil selon l'une quelconque des revendications précédentes, comprenant en outre un organe isolant (938) positionné entre l'électrode de retour (112, 936) et la borne d'électrode (58, 934), l'organe isolant (938) espaçant l'électrode de retour de la borne d'électrode pour minimiser un contact direct entre l'électrode de retour et une structure corporelle au niveau du site cible lorsque la borne d'électrode est positionnée à proximité étroite ou en contact partiel avec la structure corporelle.

12. Appareil selon l'une quelconque des revendications précédentes, dans lequel la portion d'extrémité distale de l'arbre est configurée pour une translation axiale dans un canal formé par l'enlèvement volumétrique de tissu.

13. Appareil selon la revendication 12, comprenant en outre un moyen pour endommager thermiquement les parois internes du canal formé par l'enlèvement volumétrique de tissu sans enlever ou faire une ablation desdites parois internes.

14. Appareil selon la revendication 13, dans lequel l'électrode de retour (112) est dimensionnée et positionnée par rapport à la borne d'électrode (58) de telle sorte que, lorsque l'électrode de retour a été translatée axialement dans le canal, la différence de tension fournie par l'alimentation électrique (28) fait circuler un courant à partir de la borne d'électrode (58), à travers les parois internes du canal vers l'électrode de retour (112), de telle sorte que le courant provoque un endommagement thermique des parois internes du canal.

15. Appareil selon l'une quelconque des revendications précédentes, dans lequel l'arbre est rigide.

16. Appareil selon l'une quelconque des revendications précédentes, dans lequel l'arbre comporte un coude pour permettre de décaler la portion d'extrémité distale par rapport à la portion d'extrémité proximale.

17. Appareil selon l'une quelconque des revendications précédentes, dans lequel la borne d'électrode (104) est portée par un organe de support à un angle de 10 à 90 degrés par rapport à l'axe longitudinal de l'arbre.

18. Appareil selon l'une quelconque des revendications précédentes, comprenant en outre un élément d'aspiration destiné à aspirer le fluide du site cible.

19. Appareil selon l'une quelconque des revendications précédentes, dans lequel l'arbre comporte un élément de fourniture de fluide (14, 15) destiné à fournir un fluide au site cible, l'élément de fourniture de fluide définissant un chemin de fluide en contact électrique avec l'électrode de retour (112) et la borne d'électrode (58).

20. Appareil selon l'une quelconque des revendications précédentes, dans lequel l'électrode de retour (112) est un organe tubulaire et l'élément de fourniture de fluide comprend une lumière axiale (54) couplée à l'électrode de retour, la lumière axiale formant au moins une portion du chemin de fluide et ayant une évacuation en communication de fluide avec la borne d'électrode (58).

21. Appareil selon l'une quelconque des revendications précédentes, dans lequel le site cible comprend un cornet à l'intérieur d'une cavité nasale du nez d'un patient.
